(19) Europäisches Patentamt
European Patent Office
Office européen des brevets

(11) **EP 4 378 452 A1**

(12) **EUROPEAN PATENT APPLICATION**
published in accordance with Art. 153(4) EPC

(43) Date of publication:
**05.06.2024 Bulletin 2024/23**

(21) Application number: **22849559.4**

(22) Date of filing: **27.07.2022**

(51) International Patent Classification (IPC):
***A61K 9/107*** (2006.01) ***A61K 9/12*** (2006.01)
***A61K 38/09*** (2006.01) ***A61K 45/00*** (2006.01)
***A61K 47/14*** (2017.01) ***A61K 47/24*** (2006.01)
***A61P 13/08*** (2006.01) ***A61P 35/00*** (2006.01)

(52) Cooperative Patent Classification (CPC):
**A61K 9/107; A61K 9/12; A61K 38/09; A61K 45/00; A61K 47/14; A61K 47/24; A61P 13/08; A61P 35/00**

(86) International application number:
**PCT/JP2022/029014**

(87) International publication number:
**WO 2023/008498 (02.02.2023 Gazette 2023/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.07.2021 JP 2021122489**

(71) Applicant: **Farnex Incorporated**
**Ota-ku**
**Tokyo 145-0064 (JP)**

(72) Inventors:
- **SUGIBAYASHI Kenji**
  **Kawagoe-shi, Saitama 350-1123 (JP)**
- **TODO Hiroaki**
  **Tsurugashima-shi, Saitama 350-2206 (JP)**
- **ITAKURA Shoko**
  **Tsurugashima-shi, Saitama 350-2213 (JP)**
- **HIJIKURO Ichiro**
  **Yokohama-shi, Kanagawa 230-0045 (JP)**

(74) Representative: **Mewburn Ellis LLP**
**Aurora Building**
**Counterslip**
**Bristol BS1 6BX (GB)**

# (54) NON-LAMELLAR LIQUID CRYSTAL-FORMING COMPOSITION AND USE THEREOF

(57) The present invention provides a novel non-lamellar liquid crystal-forming composition. An aspect of the present invention is a non-lamellar liquid crystal-forming composition comprising a fatty acid ester and a phospholipid. The fatty acid ester has a hydrophilic group having one hydroxyl group or a salt thereof, and the fatty acid ester is a fatty acid ester having none of the structures represented by General Formulas (A) to (D) (where m is independently 1 or 2; a, b, c, and d are each independently a binding site to a hydrophilic group; n is 0, 1 or 2; and the wavy lines each independently mean an E-isomer or a Z-isomer).

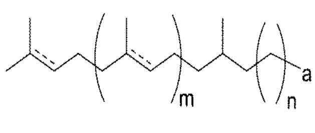

• • • (A)

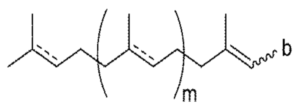

• • • (B)

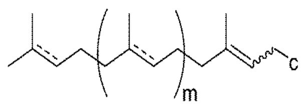

• • • (C)

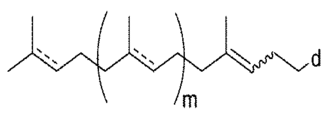

• • • (D)

EP 4 378 452 A1

Fig. 1

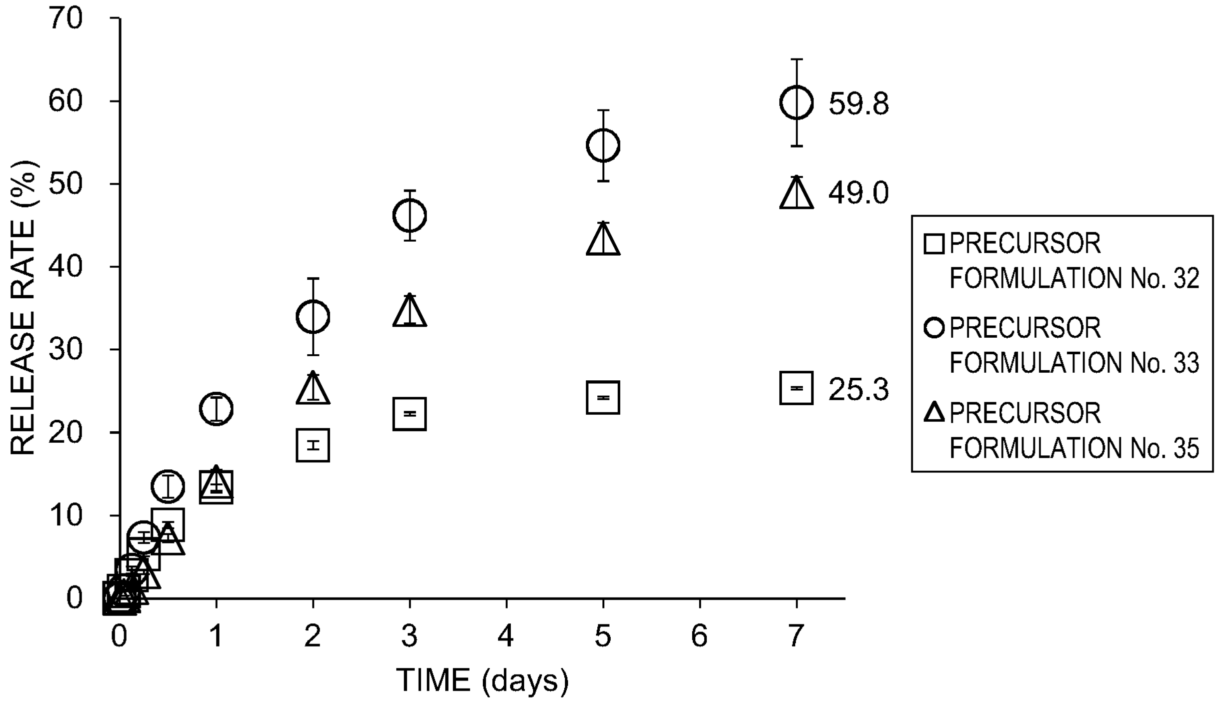
70
60
50
40
30
20
10
0
RELEASE RATE (%)
0
1
2
3
4
5
6
7
TIME (days)
59.8
49.0
25.3
PRECURSOR FORMULATION No. 32
PRECURSOR FORMULATION No. 33
PRECURSOR FORMULATION No. 35

## Description

### Technical Field

The present invention relates to a non-lamellar liquid crystal-forming composition and use thereof.

### Background Art

Many reports have mentioned the usefulness of lyotropic liquid crystals such as liposome as biomimetic drug delivery system (DDS) carriers since the DDS concept was proposed. In recent years, non-lamellar liquid crystals (NLLCs), one type of the lyotropic liquid crystals, have been reported to have advantages such as high drug content percentages and easy preparability, as compared with conventional DDS carriers.

Various liquid crystal-forming compounds have been used for various applications in the fields of cosmetics, pharmaceutical products, and the like. For example, a sustained release lipid pre-concentrate is known. The pre-concentrate comprises a sorbitan unsaturated fatty acid ester having a polar head with two or more - OH groups, a phospholipid, and a particular liquid crystal hardener. The pre-concentrate exists as a lipid liquid phase in the absence of any aqueous fluid and forms liquid crystals on an aqueous fluid (for example, see Patent Literature 1).

Another example is a pre-formulation comprising a low-viscosity, non-liquid crystalline mixture of an ester of a sugar or a sugar derivative, a phospholipid, and a biocompatible, oxygen-containing, low-viscosity organic solvent. The pre-formulation forms or is capable of forming a non-lamellar liquid crystal phase structure upon contact with an aqueous fluid, and the pre-formulation does not further contain a particular liquid crystal hardener (for example, see Patent Literature 2).

### Citation List

### Patent Literature

Patent Literature 1: Japanese Patent Publication No. 2014-527545A
Patent Literature 2: Japanese Patent Publication No. 2018-502091A

### Summary of Invention

### Technical Problem

The present invention is intended to provide a novel non-lamellar liquid crystal-forming composition. The present invention is also intended to provide a novel compound or a salt thereof capable of producing a non-lamellar liquid crystal-forming composition.

### Solution to Problem

The present inventors have conducted diligent studies and have found a non-lamellar liquid crystal-forming composition comprising a certain fatty acid ester and a phospholipid. The present inventors have further successfully synthesized a novel compound that can be used to prepare a non-lamellar liquid crystal-forming composition. The present inventors have therefore completed the present invention.

Specifically, the present invention encompasses the following aspects.

[1] A non-lamellar liquid crystal-forming composition comprising a fatty acid ester and a phospholipid, wherein

the fatty acid ester has a hydrophilic group having one hydroxyl group or a salt thereof, and
the fatty acid ester is a fatty acid ester having none of the structures represented by General Formulas (A) to (D):

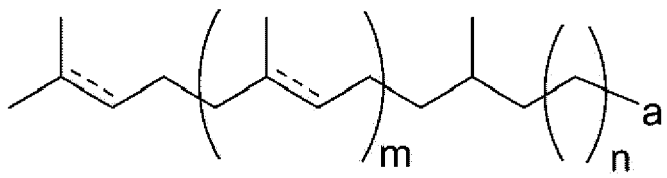

· · · (A)

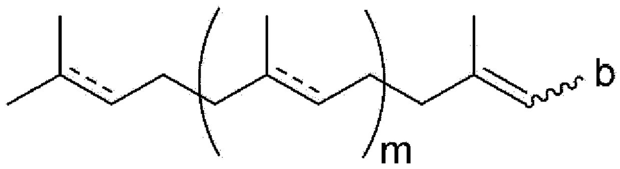

· · · (B)

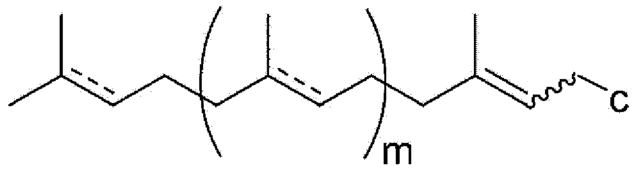

· · · (C)

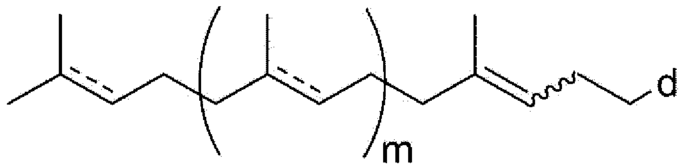

· · · (D)

in General Formulas (A) to (D), m is independently 1 or 2; a, b, c, and d are each independently a binding site to a hydrophilic group;

independently means a single bond or a double bond; in General Formula (A), n is 0, 1, or 2; and in General Formulas (B), (C), and (D), a wavy line independently means an E-isomer or a Z-isomer.

[2] The composition according to the aspect [1], wherein the fatty acid ester has a fatty acid having 6 to 24 carbon atoms.
[3] The composition according to the aspect [1] or [2], wherein the weight ratio between the fatty acid ester and the phospholipid is 90: 10 to 20:80.
[4] The composition according to any one of the aspects [1] to [3], wherein the fatty acid ester is a fatty acid ester of at least one selected from a saturated or unsaturated linear fatty acid and a derivative thereof and at least one selected from a glycol and a derivative thereof.
[5] The composition according to the aspect [4], wherein the linear fatty acid is a linear fatty acid having an unsaturation degree of 0 to 6.
[6] The composition according to the aspect [4] or [5], wherein the linear fatty acid is a linear fatty acid represented by General Formula (I):

$H_3C\text{-}R\text{-}COOH$ ... (I)

in General Formula (I), R is a linear hydrocarbon group represented by $-C_pH_q-$; p is an integer of 4 to 22; and q is an integer of 2p - 4 to 2p when p is 5 or less, is an integer of 2p - 6 to 2p when p is 6 or 7, is an integer of 2p - 8 to 2p when p is 8 or 9, is an integer of 2p - 10 to 2p when p is 10 or 11, or is an integer of 2p - 12 to 2p when p is not less than 12.
[7] The composition according to any one of the aspects [4] to [6], wherein the linear fatty acid is at least one fatty acid selected from caprylic acid, lauric acid, myristic acid, palmitic acid, oleic acid, linoleic acid, and behenic acid.

[8] The composition according to any one of the aspects [4] to [7], wherein the glycol is at least one glycol selected from propylene glycol, ethylene glycol, butylene glycol, 3-methyl-1,3-butanediol, diethylene glycol, and isosorbide.
[9] The composition according to any one of the aspects [1] to [8], wherein the phospholipid is at least one phospholipid selected from phosphatidylcholine, phosphatidylethanolamine, and a salt thereof.
[10] The composition according to any one of the aspects [1] to [9], wherein the phospholipid is at least one phospholipid selected from soybean phosphatidylcholine, egg yolk phosphatidylcholine, dimyristoyl phosphatidylcholine, dioleyl phosphatidylcholine, dioleyl phosphatidylethanolamine, and a salt thereof.
[11] The composition according to any one of the aspects [1] to [10], further comprising at least one of an oil and an organic solvent.
[12] The composition according to any one of the aspects [1] to [11], wherein the composition further comprises an aqueous medium and is a non-lamellar liquid crystal composition.
[13] The composition according to the aspect [12], wherein the composition further comprises a surfactant and is a non-lamellar liquid crystal emulsion composition.
[14] The composition according to any one of the aspects [1] to [11], wherein the composition is a liquid crystal precursor composition capable of forming a non-lamellar liquid crystal in the presence of an aqueous medium.
[15] A pharmaceutical formulation comprising the composition according to any one of the aspects [1] to [14].
[16] The pharmaceutical formulation according to the aspect [15] for adhesion prevention of living tissue.
[17] The pharmaceutical formulation according to the aspect [15], wherein the composition further comprises a drug and is a sustained release formulation.
[18] The pharmaceutical formulation according to the aspect [17], wherein the drug is a gonadotropin-releasing hormone (GnRH) agonist.
[19] The pharmaceutical formulation according to the aspect [18], wherein the GnRH agonist is leuprolide or a salt thereof.
[20] The pharmaceutical formulation according to any one of the aspects [15] to [19], wherein the pharmaceutical formulation is a spray formulation, an aerosol formulation, an injection, or a depot formulation.
[21] A compound represented by Formula (X') or a salt thereof:

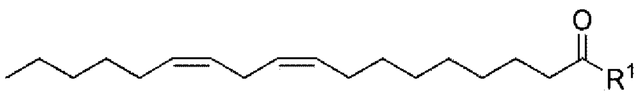

・・・ (X')

in General Formula (X'), $R^1$ is a structure derived from propylene glycol, butylene glycol, isoprene glycol, diethylene glycol, or isosorbide.
[22] A compound represented by Formula (X), (Y), or (Z):

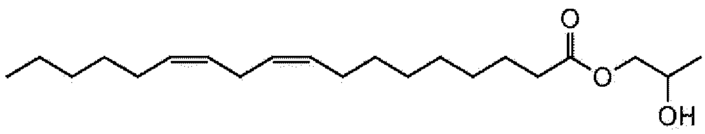

・・・ (X)

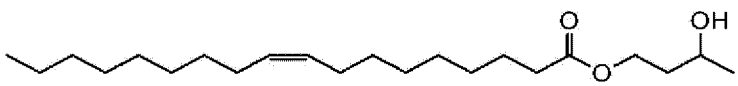

・・・ (Y)

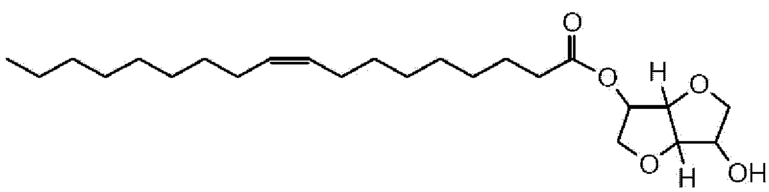

・・・ (Z)

or a salt thereof.

The present application encompasses the contents disclosed in Japanese Patent Application No. 2021-122489 on which the priority of the present application is based.

## Advantageous Effects of Invention

The present invention can provide a novel composition capable of forming non-lamellar liquid crystals.

## Brief Description of Drawings

FIG. 1 shows the release rate of a drug (leuprolide acetate) from precursor formulations No. 32, No. 33, and No. 35 containing leuprolide acetate in Example 5 (in vitro release data).
FIG. 2 shows the pharmacokinetic data (leuprolide acetate) of precursor formulations No. 34 and No. 35 containing leuprolide acetate in Example 6.
FIG. 3 shows a photograph of the administration site of a rat to which a precursor formulation No. 35 in Example 6 was subcutaneously administered.

## Description of Embodiments

The present invention will now be described in detail.
An embodiment of the present invention relates to a non-lamellar liquid crystal-forming composition comprising a fatty acid ester and a phospholipid. The fatty acid ester has a hydrophilic group having one hydroxyl group or a salt thereof, and the fatty acid ester is a fatty acid ester having none of the structures represented by General Formulas (A) to (D). The non-lamellar liquid crystal-forming composition pertaining to the present invention comprises a phospholipid and thus has excellent biocompatibility. Such a non-lamellar liquid crystal-forming composition is highly safe and can be used in the in vivo application.
The fatty acid ester used in the present invention has a hydrophilic group having one hydroxyl group or a salt thereof. The fatty acid ester used in the present invention is a fatty acid ester having none of the structures represented by General Formulas (A) to (D). A fatty acid ester having a hydrophilic group is also called an amphiphilic compound. As the fatty acid ester, a single fatty acid ester may be used or two or more fatty acid esters may be used.

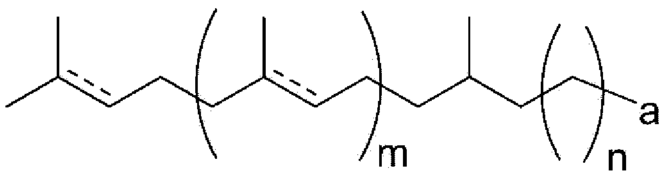

・・・(A)

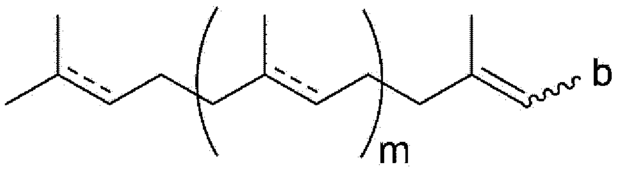

・・・(B)

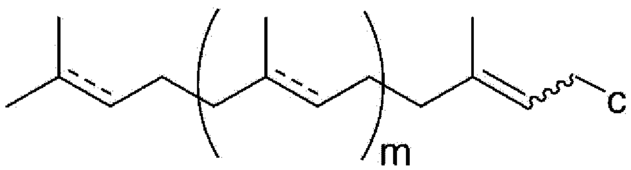

・・・(C)

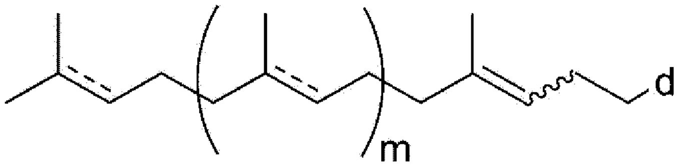

· · · (D)

In General Formulas (A) to (D), m is independently 1 or 2; a, b, c, and d are each independently the binding site to a hydrophilic group;

independently means a single bond or a double bond; in General Formula (A), n is 0, 1, or 2; and in General Formulas (B), (C), and (D), a wavy line independently means an E-isomer or a Z-isomer.

The structures represented by General Formulas (A) to (D) are what are called isoprenoid fatty acid chains. The fatty acid ester used in the present invention does not have what is called an isoprenoid fatty acid chain as a characteristic. The binding site to a hydrophilic group also means the bond to a hydrophilic group.

The fatty acid ester has a hydrophilic group having one hydroxyl group or a salt thereof. The hydrophilic group is typically a hydrophilic group comprising a carbonyl (C=O) structure derived from the fatty acid constituting a fatty acid ester and a structure derived from a compound (e.g., glycol) bonded to the fatty acid through an ester bond. The hydrophilic group has one hydroxyl group or one salt thereof. Examples of the hydroxyl group salt include metal salts (such as an alkali metal salt, an alkaline earth metal salt, and a transition metal salt) of a hydroxyl group. Preferred examples of the hydroxyl group salt include an alkali metal salt of a hydroxyl group and an alkaline earth metal salt of a hydroxyl group. Particularly preferred examples include a sodium salt of a hydroxyl group (-ONa), a potassium salt of a hydroxyl group (-OK), a calcium salt of a hydroxyl group ($-OCa_{1/2}$), and a magnesium salt of a hydroxyl group ($-OMg_{1/2}$). The salt of a hydroxyl group may be a pharmaceutically acceptable salt other than the above. The present inventors have found that using a fatty acid ester having a hydrophilic group with one hydroxyl group or a salt thereof as the fatty acid ester can reduce the viscosity of a composition as compared with when a fatty acid ester having a hydrophilic group with a plurality of hydroxyl groups or salts thereof is used. When a composition having a low viscosity is used, for example, as an injection, a thinner injection needle can be used advantageously. A composition having a low viscosity is more easily prepared than a composition having a high viscosity and is likely to be highly stable in a wide range of temperatures. Moreover, a composition containing a fatty acid ester in a wide range of weight ratios can be prepared as a liquid composition and thus is preferred as the composition.

The carbon number of the fatty acid of the fatty acid ester, that is, the carbon number of the fatty acid constituting the fatty acid ester (the fatty acid as the material of the fatty acid ester) is preferably 6 to 24, more preferably 8 to 22, and even more preferably 8 to 18. Within the range, a non-lamellar liquid crystal-forming composition can be easily prepared, and thus such a carbon number is preferred. The fatty acid may be a branched fatty acid. As the branched fatty acid, a saturated or unsaturated branched fatty acid may be used. Examples of the branched fatty acid include 2-ethylhexanoic acid, isononanoic acid, isodecanoic acid, isotridecanoic acid, isomyristic acid, isopalmitic acid, isostearic acid, and isoicosanoic acid.

In a preferred embodiment, the fatty acid is a saturated or unsaturated linear fatty acid. The fatty acid may be a synthetic fatty acid, a hemisynthetic fatty acid, or a natural fatty acid. In a preferred embodiment, the fatty acid is a natural fatty acid. In a preferred embodiment, the fatty acid ester is a fatty acid ester of at least one selected from a fatty acid and a derivative thereof and at least one selected from a glycol and a derivative thereof.

In other words, in a preferred embodiment, the fatty acid ester is a fatty acid ester of at least one selected from a saturated or unsaturated linear fatty acid and a derivative thereof and at least one selected from a glycol and a derivative thereof. The fatty acid ester may be produced by any method. For example, at least one selected from a saturated or unsaturated linear fatty acid and a derivative thereof is reacted with at least one selected from a glycol and a derivative thereof under a known condition, and accordingly, the carboxyl group derived from the fatty acid is reacted with the hydroxyl group derived from the glycol to form an ester bond, giving an intended fatty acid ester. As a specific example, the method in Examples described later can be used to produce a fatty acid ester.

When the linear fatty acid is an unsaturated fatty acid, the unsaturation degree, that is, the number of carbon-carbon double bonds is typically 1 or more, preferably 1 to 6, more preferably 1 to 4, and particularly preferably 1 or 2. In other words, the linear fatty acid is preferably a linear fatty acid having an unsaturation degree of 0 to 6, more preferably a linear fatty acid having an unsaturation degree of 0 to 4, and particularly preferably a linear fatty acid having an unsaturation degree of 0 to 2. When the linear fatty acid is an unsaturated fatty acid having an unsaturation degree of 1 or 2, the carbon number is 18 or less from the viewpoint of the stability of the composition in a preferred embodiment. When the linear fatty acid is a saturated fatty acid, the carbon number is 16 or less from the viewpoint of the stability of

the composition in a preferred embodiment.

In a preferred embodiment, the linear fatty acid is a linear fatty acid represented by General Formula (I). In other words, in a preferred embodiment, the linear fatty acid has a methyl group at one terminal.

$$H_3C\text{-}R\text{-}COOH \ldots \qquad (I)$$

In General Formula (I), R is a linear hydrocarbon group represented by $-C_pH_q-$; p is an integer of 4 to 22; and q is an integer of 2p - 4 to 2p when p is 5 or less, is an integer of 2p - 6 to 2p when p is 6 or 7, is an integer of 2p - 8 to 2p when p is 8 or 9, is an integer of 2p - 10 to 2p when p is 10 or 11, or is an integer of 2p - 12 to 2p when p is not less than 12.

p is more preferably an integer of 6 to 20 and even more preferably 6 to 16. Regardless of the value of p, q is 2p - 4 to 2p in a preferred embodiment. When q is 2p - 12, the linear hydrocarbon group represented by $-C_pH_q-$ has an unsaturation degree of 6. When q is 2p - 10, the linear hydrocarbon group represented by $-C_pH_q-$ has an unsaturation degree of 5. When q is 2p - 8, the linear hydrocarbon group represented by $-C_pH_q-$ has an unsaturation degree of 4. When q is 2p - 6, the linear hydrocarbon group represented by $-C_pH_q-$ has an unsaturation degree of 3. When q is 2p - 4, the linear hydrocarbon group represented by $-C_pH_q-$ has an unsaturation degree of 2. When q is 2p - 2, the linear hydrocarbon group represented by $-C_pH_q-$ has an unsaturation degree of 1. When q is 2p, the linear hydrocarbon group represented by $-C_pH_q-$ is a saturated linear hydrocarbon group (a linear alkylene group having an unsaturation degree of 0). The above 2p - 4 to 2p means 2p - 4, 2p - 2, or 2p. The above 2p - 6 to 2p means 2p - 6, 2p - 4, 2p - 2, or 2p. The above 2p - 8 to 2p means 2p - 8, 2p - 6, 2p - 4, 2p - 2, or 2p. The above 2p - 10 to 2p means 2p - 10, 2p - 8, 2p - 6, 2p - 4, 2p - 2, or 2p. The above 2p - 12 to 2p means 2p - 12, 2p - 10, 2p - 8, 2p - 6, 2p - 4, 2p - 2, or 2p.

Examples of the fatty acid include butanoic acid, pentanoic acid, caproic acid, enanthic acid, caprylic acid, pelargonic acid, capric acid, lauric acid, myristic acid, pentadecylic acid, palmitic acid, palmitoleic acid, margaric acid, stearic acid, oleic acid, vaccenic acid, linoleic acid, (9,12,15)-linolenic acid, (6,9,12)-linolenic acid, eleostearic acid, arachidic acid, 8,11-eicosadienoic acid, mead acid, arachidonic acid, behenic acid, eicosapentaenoic acid, docosahexaenoic acid, lignoceric acid, and nervonic acid. Fatty acids may be used singly or in combination of two or more of them. As the fatty acid, a linear fatty acid is preferred. Specifically preferred is at least one fatty acid selected from caprylic acid, lauric acid, myristic acid, palmitic acid, oleic acid, linoleic acid, and behenic acid. Fatty acids may be used singly or in combination of two or more of them. In some cases, a commercially available fatty acid can be a mixture of fatty acids, and such a fatty acid may also be used without problems.

The glycol means a chain or cyclic compound consisting of carbons, oxygens, and hydrogens and having two hydroxyl groups bonded to different two carbon atoms. The glycol is preferably at least one glycol selected from propylene glycol, ethylene glycol, butylene glycol, 3-methyl-1,3-butanediol, diethylene glycol, and isosorbide.

When two hydroxyl groups of a glycol are nonequivalent, for example, when one hydroxyl group of a glycol is a primary hydroxyl group and the other is a secondary hydroxyl group, a resulting fatty acid ester includes isomers in which different hydroxyl groups are bonded to the fatty acid. The fatty acid ester used in the present invention may be one of such isomers or a mixture of both isomers. Typically, when a glycol having a primary hydroxyl group and a secondary hydroxyl group is used to synthesize a fatty acid ester, a fatty acid ester in which the primary hydroxyl group is bonded to the fatty acid is thought to be contained at a higher ratio than a fatty acid ester in which the secondary hydroxyl group is bonded to the fatty acid. In the examples, the chemical formula of a synthesized fatty acid ester is shown but is merely a typical structure, and the presence of isomers in which a different hydroxyl group of a glycol is bonded to a fatty acid is not denied.

The case in which a glycol has two hydroxyl groups that are nonequivalent includes cases in which propylene glycol, 1,3-butylene glycol, 3-methyl-1,3-butanediol, isosorbide, and the like are used.

Examples of the derivative of a saturated or unsaturated linear fatty acid include an alkyl ester of the above saturated or unsaturated linear fatty acid (an ester of the saturated or unsaturated linear fatty acid and a monohydric alcohol) and a salt of the saturated or unsaturated linear fatty acid (such as a metal salt). Examples of the derivative of a glycol include an ether of the above glycol and a monool (such as a glycol monoether in which one hydroxyl group of a glycol is etherified with a monool) and a salt of the glycol (such as a metal salt). Derivatives of the saturated or unsaturated linear fatty acid may be used singly or in combination of two or more of them. In some cases, a commercially available derivative of a saturated or unsaturated linear fatty acid can be a mixture of a plurality of components (a mixture of multiple types of derivatives of a saturated or unsaturated linear fatty acid), and such derivatives of a saturated or unsaturated linear fatty acid may also be used without problems.

Preferred examples of the fatty acid ester used in the present invention include, but are not limited to,

propylene glycol monocaprylate,
propylene glycol monooleate,
propylene glycol monomyristate,
propylene glycol monopalmitate,

propylene glycol monolinoleate,
propylene glycol monobehenate,
ethylene glycol monocaprylate,
ethylene glycol monooleate,
ethylene glycol monomyristate,
ethylene glycol monopalmitate,
ethylene glycol monolinoleate,
ethylene glycol monobehenate,
1,3-butylene glycol monocaprylate,
1,3-butylene glycol monooleate,
1,3-butylene glycol monomyristate,
1,3-butylene glycol monopalmitate,
1,3-butylene glycol monolinoleate,
1,3-butylene glycol monobehenate,
3-methyl-1,3-butanediol monocaprylate,
3-methyl-1,3-butanediol monooleate,
3-methyl-1,3-butanediol monomyristate,
3-methyl-1,3-butanediol monopalmitate,
3-methyl-1,3-butanediol monolinoleate,
3-methyl-1,3-butanediol monobehenate,
diethylene glycol monocaprylate,
diethylene glycol monooleate,
diethylene glycol monomyristate,
diethylene glycol monopalmitate,
diethylene glycol monolinoleate,
diethylene glycol monobehenate,
isosorbide monocaprylate,
isosorbide monooleate,
isosorbide monomyristate,
isosorbide monopalmitate,
isosorbide monolinoleate, and
isosorbide monobehenate. Other examples include a salt of a hydroxyl group of a hydrophilic group of the above fatty acid ester. Preferred examples of the salt of a hydroxyl group include a sodium salt of a hydroxyl group (-ONa), a potassium salt of a hydroxyl group (-OK), a calcium salt of a hydroxyl group ($-OCa_{1/2}$), a magnesium salt of a hydroxyl group ($-OMg_{1/2}$), and other pharmaceutically acceptable salts.

Particularly preferred examples of the fatty acid ester used in the present invention include, but are not limited to,

propylene glycol monooleate,
propylene glycol monolinoleate,
ethylene glycol monooleate,
ethylene glycol monolinoleate,
1,3-butylene glycol monooleate,
1,3-butylene glycol monolinoleate,
3-methyl-1,3-butanediol monooleate,
3-methyl-1,3-butanediol monolinoleate,
diethylene glycol monooleate,
diethylene glycol monolinoleate,
isosorbide monooleate, and
isosorbide monolinoleate. Other examples include a salt of a hydroxyl group of a hydrophilic group of the above fatty acid ester. Preferred examples of the salt of a hydroxyl group include a sodium salt of a hydroxyl group (-ONa), a potassium salt of a hydroxyl group (-OK), a calcium salt of a hydroxyl group ($-OCa_{1/2}$), a magnesium salt of a hydroxyl group ($-OMg_{1/2}$), and other pharmaceutically acceptable salts.

The non-lamellar liquid crystal-forming composition pertaining to the present invention may comprise one or two or more of the above fatty acid esters. When the non-lamellar liquid crystal-forming composition pertaining to the present invention comprises a plurality of the above fatty acid esters, the weight ratio between these fatty acid esters is not particularly limited.

Of the fatty acid esters used in the present invention, the compound represented by Formula (X') is a novel compound, and the compound represented by Formula (X') or a salt thereof is exemplified as one of the present embodiment.

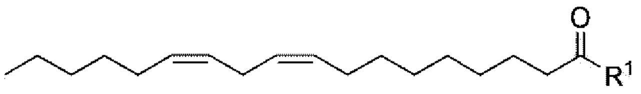
• • • (X')

In General Formula (X'), $R^1$ is a structure derived from propylene glycol, butylene glycol, isoprene glycol, diethylene glycol, or isosorbide.

The compound represented by Formula (X') is a fatty acid ester of linoleic acid and propylene glycol, butylene glycol, isoprene glycol (3-methyl-1,3-butanediol), diethylene glycol, or isosorbide. In other words, the structure derived from propylene glycol, butylene glycol, isoprene glycol, diethylene glycol, or isosorbide means a structure in which the hydrogen atom of one hydroxyl group is removed from propylene glycol, butylene glycol, isoprene glycol, diethylene glycol, or isosorbide. As an example, when $R^1$ is a structure derived from diethylene glycol, the structure is $-O-CH_2-CH_2-O-CH_2-CH_2-OH$.

Of the fatty acid esters used in the present invention, the compound represented by Formula (X), (Y), or (Z) is a novel compound, and the compound represented by Formula (X), (Y), or (Z) or a salt thereof is exemplified as one of the present embodiment.

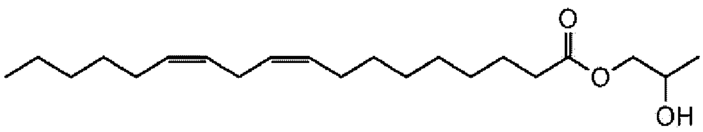
• • • (X)

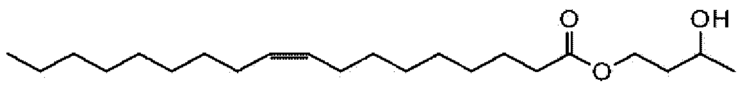
• • • (Y)

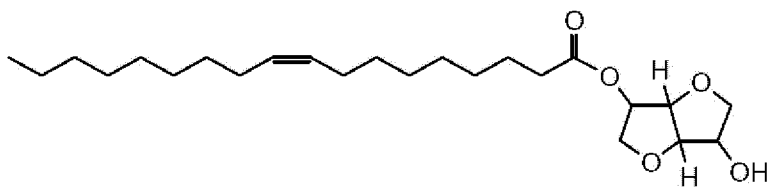
• • • (Z)

The compound represented by Formula (X) is propylene glycol monolinoleate (propylene glycol monolinoleate (C18:2)), the compound represented by Formula (Y) is 1,3-butylene glycol monooleate (butylene glycol monooleate (C18:1)), and the compound represented by Formula (Z) is isosorbide monooleate.

Examples of the salt of the compound represented by Formula (X'), (X), (Y), or (Z) include a salt of a hydroxyl group in the molecule and specifically include an alkali metal salt of a hydroxyl group and an alkaline earth metal salt of a hydroxyl group. Particularly preferred examples include a sodium salt of a hydroxyl group (-ONa), a potassium salt of a hydroxyl group (-OK), a calcium salt of a hydroxyl group ($-OCa_{1/2}$), and a magnesium salt of a hydroxyl group ($-OMg_{1/2}$). The salt of a hydroxyl group may be other pharmaceutically acceptable salts.

In the present invention, the above fatty acid ester may be used in combination with a phospholipid to produce a non-lamellar liquid crystal-forming composition. In other words, the present invention provides a composition, specifically a non-lamellar liquid crystal-forming composition, comprising the fatty acid ester and a phospholipid.

The above fatty acid ester may be used in combination with a phospholipid. The fatty acid ester was not able to form non-lamellar liquid crystals by itself, with some exceptions. The present inventors have conducted diligent studies and have found that a combination of the fatty acid ester with a phospholipid enables the formation of non-lamellar liquid crystals. If the fatty acid ester has a toxicity, the phospholipid reduces the toxicity of the fatty acid ester and increases the biocompatibility of a non-lamellar liquid crystal-forming composition, and this improves the safety when the composition is applied to a living body. In the present invention, the "biocompatibility" refers to a property of causing little or no adverse reaction (side effect) in a living body in the in vivo application. Examples of the "toxicity" in the context of the present invention include, but are not limited to, systemic toxicities such as hepatotoxicity and local toxicities that cause foreign body reactions such as abscess development or tissue damages such as bleeding or discoloration. The hepatotoxicity can be identified by the occurrence of at least one symptom that manifests liver damage, selected from occurrence of ascites, enlargement of the liver, perihepatic adhesions (particularly, adhesions at a noninjured or non-inflammatory site), whitening of the liver, and the like. The non-lamellar liquid crystal-forming composition comprising a fatty acid ester and a phospholipid pertaining to the present invention can be used in the in vivo application (preferably, parenteral administration such as intraperitoneal administration, intramuscular administration, or subcutaneous administration). In the present invention, the phrase "capable of being used in the in vivo application" means that administration to a living body (typically, parenteral administration such as intraperitoneal administration, intramuscular administration, or subcutaneous administration to the body) causes no toxicity or causes a low toxicity at a pharmaceutically acceptable level. However, the non-lamellar liquid crystal-forming composition pertaining to the present invention is not limited to the in vivo application.

Examples of the phospholipid used in the present invention include, but are not limited to, one or two or more phospholipids selected from the group consisting of phosphatidylcholine, phosphatidylethanolamine, phosphatidylserine, phosphatidylinositol, phosphatidylglycerin, phosphatidic acid, sphingomyelin, and salts thereof; a phospholipid formulation containing the phospholipid; and a fraction containing the phospholipid. Examples of the phosphatidylcholine include, but are not limited to, dioleyl phosphatidylcholine (DOPC), dimyristoyl phosphatidylcholine (DMPC), dipalmitoyl phosphatidylcholine (DPPC), soybean phosphatidylcholine (SPC; also called soybean lecithin), and egg yolk phosphatidylcholine (EPC; also called egg yolk lecithin). Examples of the phosphatidylethanolamine include, but are not limited to, dioleyl phosphatidylethanolamine (DOPE). Examples of the phosphatidylglycerin include, but are not limited to, dioleyl phosphatidylglycerin, and examples of the phosphatidylglycerin salt include, but are not limited to, sodium dioleyl phosphatidylglycerin (DOPG-Na). The phospholipid used in the present invention may be a synthetic phospholipid or a natural phospholipid. In an embodiment, the phospholipid used in the present invention may be a phosphatidylcholine and may be, for example, soybean phosphatidylcholine or egg yolk phosphatidylcholine. In another embodiment, the phospholipid used in the present invention may be phosphatidylcholine, phosphatidylethanolamine, phosphatidylglycerin, or a salt thereof. In an embodiment, the phospholipid used in the present invention may be a phospholipid selected from the group consisting of soybean phosphatidylcholine (SPC), egg yolk phosphatidylcholine (EPC), dimyristoyl phosphatidylcholine (DMPC), dioleyl phosphatidylcholine (DOPC), and dioleyl phosphatidylethanolamine (DOPE). In a preferred embodiment, the phospholipid may be at least one phospholipid selected from phosphatidylcholine, phosphatidylethanolamine, and a salt thereof. In another preferred embodiment, the phospholipid may be at least one phospholipid selected from soybean phosphatidylcholine, egg yolk phosphatidylcholine, dimyristoyl phosphatidylcholine, dioleyl phosphatidylcholine, dioleyl phosphatidylethanolamine, and a salt thereof. The non-lamellar liquid crystal-forming composition pertaining to the present invention may comprise one or more phospholipids.

In order to increase the biocompatibility of the amphiphilic compound to improve the safety of the non-lamellar liquid crystal-forming composition, the weight ratio between the above fatty acid ester and the above phospholipid contained in the non-lamellar liquid crystal-forming composition pertaining to the present invention, the fatty acid ester:the phospholipid, is preferably 90:10 to 10:90, more preferably 90:10 to 20:80, and particularly preferably 80:20 to 20:80, but the ratio is not limited to them. As other examples, the fatty acid ester:the phospholipid may be 80:20 to 30:70, 70:30 to 10:90, 70:30 to 20:80, 70:30 to 30:70, 60:40 to 10:90, 60:40 to 20:80, 60:40 to 30:70, 60:40 to 40:60, 45:55 to 10:90, 45:55 to 20:80, 45:55 to 30:70, 45:55 to 35:65, 45:55 to 55:45, 50:50 to 20:80, 50:50 to 30:70, 40:60 to 10:90, 40:60 to 20:80, 40:60 to 30:70, 35:65 to 20:80, or 35:65 to 25:75.

When a plurality of the fatty acid esters are used, the weight ratio between the fatty acid esters and the phospholipid is calculated by using the total amount (weight) of the fatty acid esters. When a plurality of the phospholipids are used, the weight ratio is calculated by using the total amount (weight) of the phospholipids. In the description, the term "weight" is used interchangeably with the term "mass".

In an alternative embodiment, the non-lamellar liquid crystal-forming composition pertaining to the present invention may comprise no phospholipid, depending on the application, the dosage form, the composition, or the like, as long as the composition has sufficiently high biocompatibility (i.e., safety) and has non-lamellar liquid crystal-forming properties. Hence, the present invention also relates to a composition, specifically a non-lamellar liquid crystal-forming composition, comprising the above fatty acid ester. In the alternative embodiment, a fatty acid ester that is produced from a glycol having no ether bond and has a hydrophilic group having one hydroxyl group or a salt thereof is difficult to form non-lamellar liquid crystals without any phospholipid and thus cannot be used, in some cases. In contrast, a fatty acid ester that is produced from a glycol having an ether bond and has a hydrophilic group having one hydroxyl group or a salt thereof, such as diethylene glycol monooleate, is likely to be suitably usable in the alternative embodiment. When, for example, containing a drug, such a non-lamellar liquid crystal-forming composition may be used as a sustained release formulation.

In the present invention, the "non-lamellar liquid crystal-forming composition" means a composition having a non-lamellar liquid crystal structure (a non-lamellar liquid crystal composition) or a composition that forms no non-lamellar liquid crystal structure by itself but is capable of forming a non-lamellar liquid crystal structure in the presence of water (i.e., by contact with an aqueous medium) (liquid crystal precursor composition).

When the non-lamellar liquid crystal-forming composition pertaining to the present invention is a liquid crystal precursor composition, the composition comprises no aqueous medium or comprises an aqueous medium in an amount insufficient to form a non-lamellar liquid crystal structure. In other words, in an embodiment, the non-lamellar liquid crystal-forming composition pertaining to the present invention is a liquid crystal precursor composition capable of forming non-lamellar liquid crystals in the presence of an aqueous medium. In a more specific embodiment, the composition is a liquid crystal precursor composition comprising no aqueous medium and capable of forming non-lamellar liquid crystals in the presence of an aqueous medium. When the non-lamellar liquid crystal-forming composition pertaining to the present invention is a non-lamellar liquid crystal composition, the composition comprises an aqueous medium and preferably comprises an aqueous medium in an amount sufficient to form a non-lamellar liquid crystal structure. The aqueous medium is not specifically limited and may be sterile water, purified water, distilled water, ion-exchanged water, ultrapure water, water for injection, physiological saline, a phosphate buffer, or the like. The non-lamellar liquid crystal-forming composition pertaining to the present invention may be a liquid crystal emulsion (more specifically, a non-lamellar liquid crystal emulsion composition). The non-lamellar liquid crystal-forming composition pertaining to the present invention as a liquid crystal emulsion preferably further comprises a surfactant. The non-lamellar liquid crystal emulsion composition is also called a dispersion. The non-lamellar liquid crystal emulsion composition pertaining to the present invention comprising the amphiphilic compound and the phospholipid exhibits high stability.

Examples of the surfactant used in the non-lamellar liquid crystal-forming composition pertaining to the present invention include P80 (polyoxyethylene sorbitan monooleate (20E.O.)). Other examples of the surfactant include nonionic surfactants including block copolymers of hydrophilic ethylene oxide and hydrophobic propylene oxide (polyoxyethylene polyoxypropylene glycol), polyoxyethylene alkyl ether, polyoxyethylene alkyl ester, and polyoxyethylene hydrogenated castor oil. The nonionic surfactant is more preferably a nonionic surfactant having a molecular weight of 1,000 or more (more preferably, 5,000 or more). Examples of the block copolymer of ethylene oxide and propylene oxide include polyoxyethylene(200) polyoxypropylene(70) glycol, polyoxyethylene(196) polyoxypropylene(67) glycol, polyoxyethylene(160) polyoxypropylene(30) glycol, and polyoxyethylene(120) polyoxypropylene(40) glycol. These block copolymers of ethylene oxide and propylene oxide are commercially available under various names such as Pluronic (registered trademark), Poloxamer (registered trademark), Unilube (registered trademark), and Pronon (registered trademark). Particularly preferred examples of the nonionic surfactant include polyoxyethylene(200) polyoxypropylene(70) glycol and polyoxyethylene(196) polyoxypropylene(67) glycol (also called Pluronic (registered trademark) F127, Unilube 70DP-950B, and Poloxamer (registered trademark) 407). In the present invention, the above fatty acid ester has a hydrophilic group and thus functions as an amphiphilic compound but is not included in the surfactant. The non-lamellar liquid crystal-forming composition pertaining to the present invention may comprise one or two or more such surfactants.

The non-lamellar liquid crystal-forming composition pertaining to the present invention may comprise at least one of an oil and an organic solvent.

Examples of the oil used in the non-lamellar liquid crystal-forming composition pertaining to the present invention include, but are not limited to, plant oils such as sesame oil, soybean oil, corn oil, coconut oil, safflower oil, perilla oil, olive oil, castor oil, and cottonseed oil; animal oils such as egg yolk oil, fish oil, and lanolin; mineral oils such as medium-chain triglyceride (MCT), triglyceride, and liquid paraffin; hydrocarbon oils such as squalene and squalane; ester oils such as isopropyl myristate (IPM); cholesterol, tocopherol, tocopherol acetate, glyceryl dioleate (GDO), gelled hydrocarbon, methyl tetrahydrofamesylacetate, and methyl hexahydrogeranylgeranylacetate. The oil is preferably a pharmaceutically acceptable oil.

The total amount of the fatty acid ester, the phospholipid, and the oil contained in the non-lamellar liquid crystal-forming composition pertaining to the present invention is not particularly limited. When the non-lamellar liquid crystal-forming composition pertaining to the present invention is a liquid crystal precursor composition, the total amount of the fatty acid ester, the phospholipid, and the oil is, for example, 30% or more of the amount of the whole composition, typically 60 to 100%, preferably 65 to 95%, for example, about 75 to 95%, 75 to 93%, or 80 to 95%. When the non-lamellar liquid crystal-forming composition pertaining to the present invention is a liquid crystal emulsion, the total amount of the fatty acid ester, the phospholipid, and the oil varies according to the application of the liquid crystal emulsion or the like, but is, for example, 0.01 to 40% of the amount of the whole composition, preferably about 1 to 30%, for example, 20 to 30%, 20 to 23%, or 25 to 30%.

In the present description, the percentage (%) of a component in a non-lamellar liquid crystal-forming composition means % by weight and can be expressed in units of w/w%.

Examples of the organic solvent used in the non-lamellar liquid crystal-forming composition pertaining to the present invention include, but are not limited to, alcohols such as ethanol, propylene glycol, and isopropanol; ethers such as diethyl ether and polyethylene glycol; dimethyl sulfoxide (DMSO); N-methylpyrrolidone (NMP); and dimethylacetamide (DMA). The organic solvent is preferably a pharmaceutically acceptable organic solvent. In the non-lamellar liquid crystal-forming composition pertaining to the present invention, a protic organic solvent or an aprotic organic solvent may be used singly, or a protic organic solvent and an aprotic organic solvent may be used in combination. Examples of the protic organic solvent include alcohols such as ethanol and propylene glycol; and polyethylene glycol.

Examples of the aprotic organic solvent include ethers such as diethyl ether; dimethyl sulfoxide (DMSO); N-methylpyrrolidone (NMP); and dimethylacetamide (DMA).

When the non-lamellar liquid crystal-forming composition pertaining to the present invention comprises an oil, the ratio between the total weight of the fatty acid ester and the phospholipid contained in the non-lamellar liquid crystal-forming composition and the weight of the oil, the total weight of the fatty acid ester and the phospholipid:the weight of the oil, is preferably 30:70 to 99:1, more preferably 40:60 to 98:2, and particularly preferably 45:55 to 97:3. When the non-lamellar liquid crystal-forming composition pertaining to the present invention comprises an organic solvent, the ratio between the total weight of the fatty acid ester, the phospholipid, and an optional oil contained in the non-lamellar liquid crystal-forming composition and the weight of the organic solvent, the total weight of the fatty acid ester, the phospholipid, and an optional oil:the weight of the organic solvent, is preferably 80:20 to 99:1, more preferably 85:15 to 97:3, and particularly preferably 90:10 to 95:5.

The non-lamellar liquid crystal-forming composition pertaining to the present invention may comprise a water-soluble polymer. Examples of the water-soluble polymer include, but are not limited to, hydroxypropyl cellulose (HPC), hydroxyethyl cellulose, polyvinylpyrrolidone, Carbopol, carrageenan, chitosan, chondroitin sulfate salt, xanthan gum, hyaluronic acid salts (such as sodium hyaluronate), alginic acid salts (such as sodium alginate), gelatin, and dextran. Examples of the hydroxypropyl cellulose (HPC) include HPCs in five grades commercially available from Nippon Soda Co., Ltd. (Japan): HPC-SSL (a molecular weight of about 40,000, a viscosity of 2 to 2.9 mPa s), HPC-SL (a molecular weight of about 100,000, a viscosity of 3 to 5.9 mPa s), HPC-L (a molecular weight of about 140,000, a viscosity of 6 to 10 mPa s), HPC-M (a molecular weight of about 620,000, a viscosity of 150 to 400 mPa·s), and HPC-H (a molecular weight of about 910,000, a viscosity of 1,000 to 4,000 mPa·s). In an embodiment, the hydroxypropyl cellulose may be a hydroxypropyl cellulose having a molecular weight of 1,000,000 or less or 800,000 or less, for example, 10,000 to 700,000 or 10,000 to 80,000.

The non-lamellar liquid crystal-forming composition pertaining to the present invention may comprise an antioxidant. Examples of the antioxidant include, but are not limited to, ascorbic acid and sodium sulfite.

The non-lamellar liquid crystal-forming composition pertaining to the present invention can form a gel when water is contained or in the presence of water. The gel formation means the formation of non-lamellar liquid crystals (liquid crystal gel). Non-lamellar liquid crystals can retain a substance such as a drug therein and can gradually release the substance. The present invention also relates to a gel comprising the non-lamellar liquid crystal-forming composition pertaining to the present invention (gel composition).

Non-lamellar liquid crystals are a liquid crystal structure that is not lamellar liquid crystals and specifically, can be, for example, cubic liquid crystals, reverse hexagonal liquid crystals (HII), an inverse micellar cubic phase (Fd3m), or a sponge phase (L3). The non-lamellar liquid crystals may include two or more types of non-lamellar liquid crystal phases.

The cubic liquid crystals can be cubic liquid crystals belonging to the crystallographic space group Ia3d (hereinafter called Ia3d cubic liquid crystals), cubic liquid crystals belonging to the crystallographic space group Pn3m (hereinafter called Pn3m cubic liquid crystals), or cubic liquid crystals belonging to the crystallographic space group Im3m (hereinafter called Im3m cubic liquid crystals).

The liquid crystal structure can be analyzed by a conventional method and can be analyzed, for example, by small-angle X-ray scattering (SAXS) measurement using the following method.

When a measurement sample is an emulsion, the sample can be placed, for example, in an X-ray capillary tube made of soda glass, quartz, or the like, and then the capillary tube can be sealed with an oxy-fuel burner and subjected to SAXS measurement. The SAXS measurement can be performed with a commercially available apparatus, and the measurement can be performed, for example, with a NANO-Viewer nano-scale X-ray structure analyzer (Rigaku Corp.).

By determining whether the following scattering peak ratio (peak interval) specific to the corresponding liquid crystal structure is observed in a result of SAXS measurement, the liquid crystal structure formed from the non-lamellar liquid crystal-forming composition pertaining to the present invention or formed from the composition in the presence of water can be identified.

Ratio of Pn3m cubic liquid crystals:

$$\sqrt{2}:\sqrt{3}:\sqrt{4}:\sqrt{6}:\sqrt{8}:\sqrt{9}:\sqrt{10}: ....$$

Ratio of Ia3d cubic liquid crystals:

$$\sqrt{3}:\sqrt{4}:\sqrt{7}:\sqrt{8}:\sqrt{10}:\sqrt{11}: ,,,,$$

Ratio of Im3m cubic liquid crystals:

$$\sqrt{2}:\sqrt{4}:\sqrt{6}:\sqrt{8}:\sqrt{10}:\sqrt{12}:\sqrt{14}: ,,,,$$

Ratio of Fd3m cubic liquid crystals:

$$\sqrt{3}:\sqrt{8}:\sqrt{11}:\sqrt{12}:\sqrt{16}:\sqrt{19}:\sqrt{24}:\sqrt{27}: ,,,,$$

Ratio specific to reverse hexagonal liquid crystals:

$$1:\sqrt{3}:2: ,,,,$$

In accordance with a method well known to a person skilled in the art, a peak value is calculated from SAXS intensity distribution data, and then the reciprocal ratio of the value is calculated from the peak value. This enables easy determination of the space group and the lattice constant.

In the SAXS measurement of a sponge phase (L3 phase), a broad scattering peak is observed.

By analyzing the scattering vector values of the peaks of a measurement sample, including the scattering vector value q1 [$nm^{-1}$] of a peak positioned on the smallest angle side, not only the type of liquid crystal phase but also the lattice spacing or the lattice constant thereof can be determined. The liquid crystal phase or the size of a unit lattice can be modified by changing the composition of the non-lamellar liquid crystal-forming composition on the basis of such analysis results. As a result, the sustained release properties (sustained release rate) of a drug from a composition or formulation (such as a precursor formulation or an emulsion) comprising the non-lamellar liquid crystal-forming composition and the drug can be controlled. By modifying the liquid crystal phases or the size of a unit lattice, for example, according to the fatty acid ester, the phospholipid, and other components as well as the ratio (weight ratio) thereof, and a sustained release rate suitable therefor can be achieved. Typically, the sustained release rate increases in the (ascending) order of an inverse micellar cubic phase (Fd3m), reverse hexagonal liquid crystals (HII), and cubic liquid crystals.

The non-lamellar liquid crystal-forming composition pertaining to the present invention may be used as a medical base material for the in vivo application. The non-lamellar liquid crystal-forming composition pertaining to the present invention has an adhesion preventing effect on living tissue and thus can be used for adhesion prevention of living tissue. Hence, the non-lamellar liquid crystal-forming composition pertaining to the present invention may be for adhesion prevention of living tissue. The non-lamellar liquid crystal-forming composition pertaining to the present invention may be applied (administered) as an adhesion preventing agent for living tissue into a living body.

When applied to living tissue at risk of adhesion, the non-lamellar liquid crystal-forming composition pertaining to the present invention can prevent living tissue adhesion. In the present invention, the "adhesion preventing effect" refers to an effect of preventing a tissue at risk of adhesion from adhering to another tissue or organ to result in difficulty in releasing and of suppressing adhesion completely or to a low level. Hence, the non-lamellar liquid crystal-forming composition pertaining to the present invention can be used for adhesion prevention of living tissue.

The adhesion preventing effect of the non-lamellar liquid crystal-forming composition pertaining to the present invention is brought about by the amphiphilic compound (fatty acid ester) contained in the non-lamellar liquid crystal-forming composition forming a coating on the applied tissue surface on the basis of the formation of lamellar liquid crystals. The formed coating prevents the tissue from coming into contact with other tissues or organs, and this reduces adhesion.

The adhesion preventing effect of the non-lamellar liquid crystal-forming composition pertaining to the present invention can be identified as follows: for example, the non-lamellar liquid crystal-forming composition is applied to a tissue incision of an animal model under laparotomy; the abdominal incision is closed; and the model is monitored over time. Specifically, an abdominal median incision (e.g., about 30 mm) is made to a rat, and an incision of about 20 mm is further made on each of the right and left upper parietal peritoneums. After complete hemostasis, the peritoneum incision is closed with continuous suture (using, e.g., Silk Suture 5-0). Then, a non-lamellar liquid crystal-forming composition sample is applied to either the right or left peritoneum incision so as to cover the sutured incision. When the non-lamellar liquid crystal-forming composition is a liquid crystal precursor composition, an aqueous medium (injectable water, etc.) may be further added to the sample applied site by spraying or the like so as to induce the liquid crystal formation. None is applied to the other peritoneum incision. Subsequently, two layers of the abdominal wall are closed. The rat is subjected to laparotomy after a certain period of time (e.g., 7 days) from the surgery, and whether adhesion is observed at the sutured incision can be evaluated.

The non-lamellar liquid crystal-forming composition may be applied by a method suitable for the dosage form

thereof. The application amount of the non-lamellar liquid crystal-forming composition in the above evaluation is typically, preferably 5 to 50 mg in terms of the amount of the amphiphilic compound.

Adhesion may be evaluated, for example, by rating the adhesion severity in accordance with the following evaluation scores.

· Grade 0: no adhesion
· Grade 1: adhesion releasable by mild traction (without tissue damage)
· Grade 2: adhesion releasable by strong traction (without tissue damage)
· Grade 3: adhesion released by strong traction with tissue damage

When a non-lamellar liquid crystal-forming composition sample applied to an incision site has a lower evaluation score than that at an incision site without application of the same animal individual, the sample can be determined to have the adhesion preventing effect.

For example, the adhesion range percentage of each incision is calculated as the ratio (%) of an adhesion length to the sutured incision of approximately 20 mm in length. Based on the adhesion range percentage, the following rating can be made: A, when the ratio of the adhesion range of the incision to which the non-lamellar liquid crystal-forming composition sample has been applied to that of the non-applied incision (i.e., adhesion range percentage on the sample-applied side / adhesion range percentage on the non-applied side $\times$ 100) is 40% or less; B+, when the ratio is 41 to 60%; B-, when the ratio is 61 to 80%; and C, when the ratio is 81% or more. In this evaluation, the rating of A or B+ is preferred as indicating the adhesion preventing effect.

In the present invention, the "adhesion prevention" can be determined when application of (treatment with) a non-lamellar liquid crystal-forming composition reduces the frequency and/or the degree of adhesion in the application site as compared with a control without treatment.

The present invention also provides a method for preventing adhesion of living tissue, comprising applying the non-lamellar liquid crystal-forming composition pertaining to the present invention to living tissue. More specifically, the present invention also provides a method for preventing tissue adhesion in an affected area, comprising applying an effective amount of the non-lamellar liquid crystal-forming composition pertaining to the present invention to an affected area of a patient, specifically a site at risk of adhesion, specifically a site at which tissue repair is expected to occur (such as an inflammatory site or an injured site in the body). Specific examples of such a site at risk of adhesion include exogenously or endogenously induced inflammatory sites in the body, wound sites such as surgical incisions, and sites at which the tissue surface has been damaged due to artificial treatment including contact during surgery or the like. In the present invention, the "injured site" refers to a site of a tissue or organ damaged by surgery, trauma, diseases, or the like. Examples of the tissue or organ to which the adhesion preventing agent is applied include, but are not limited to, the peritoneum, the small intestine, the large intestine, the rectum, the stomach, the duodenum, the cecum, the livers, the uterus, the uterine tubes, lymphatic vessels, the heart, the pericardium, the lungs, the brain, the ovaries, and tendons. In a typical example, the non-lamellar liquid crystal-forming composition pertaining to the present invention is applied to an incision site, the periphery of an incision site, or the whole organ with an incision site, upon surgery. The non-lamellar liquid crystal-forming composition pertaining to the present invention may be applied to a site in the body in contact with a wound site, an inflammatory site, or the like.

The application to an affected area such as an injured site (such as a wound site) or an inflammatory site may be performed by a method suitable for the dosage form. For example, the adhesion preventing agent may be sprayed onto an affected area such as an injured site (e.g., a wound site) or an inflammatory site by using a gas propellant aerosol container. Alternatively, for a pump spray formulation, the non-lamellar liquid crystal-forming composition may be sprayed onto an affected area such as an injured site (e.g., a wound site) or an inflammatory site by using a general-use non-gas propellant (e.g., manual) spray container, for example. For endoscopic surgery or laparoscopic surgery, the non-lamellar liquid crystal-forming composition may be sprayed onto an affected area such as an injured site (e.g., a wound site) by using a spray nozzle used during endoscopic surgery or laparoscopic surgery, for example. In the present invention, the "spraying" refers to ejecting (atomizing and/or squirting) an intended substance in the form of droplets, mist, fine particles, foam, or the like under pressure. When the non-lamellar liquid crystal-forming composition is a topical formulation, an appropriate amount of the topical formulation may be taken and applied to an affected area such as an injured site (e.g., a wound site) or an inflammatory site. When the non-lamellar liquid crystal-forming composition is an injection, the non-lamellar liquid crystal-forming composition may be injected into an affected area such as an injured site (e.g., a wound site) or an inflammatory site.

The non-lamellar liquid crystal-forming composition pertaining to the present invention is preferably applied to an affected area such as an injured site (e.g., a wound site) or an inflammatory site in an amount sufficient to cover the affected area such as an injured site (e.g., a wound site) or an inflammatory site. In a preferred embodiment, the specific application amount of the non-lamellar liquid crystal-forming composition pertaining to the present invention is 10 mg to 100 g, more preferably 50 mg to 50 g, and particularly preferably 0.1 g to 10 g for a human.

When the non-lamellar liquid crystal-forming composition pertaining to the present invention contains a sufficient amount of an aqueous medium (e.g., when the composition is a liquid crystal emulsion), the non-lamellar liquid crystal-forming composition can form non-lamellar liquid crystals on the applied tissue surface. When the non-lamellar liquid crystal-forming composition pertaining to the present invention contains an insufficient amount of an aqueous medium (e.g., when the composition is a liquid crystal precursor composition), non-lamellar liquid crystals are formed due to water in the body, but in order to promote the coating formation, an aqueous medium is preferably applied in addition to the non-lamellar liquid crystal-forming composition to an affected area such as an injured site (e.g., a wound site) or an inflammatory site. The aqueous medium may be, for example, water such as sterile water, purified water, distilled water, ion-exchanged water, ultrapure water, or water for injection or may be a physiologically acceptable aqueous solution. Examples of the physiologically acceptable aqueous solution include physiological saline; aqueous electrolyte solutions such as an aqueous sodium chloride solution, an aqueous calcium chloride solution, an aqueous magnesium chloride solution, an aqueous sodium sulfate solution, an aqueous potassium sulfate solution, an aqueous sodium carbonate solution, and an aqueous sodium acetate solution; buffer solutions such as a phosphate buffer and a tris-hydrochloric acid buffer; aqueous solutions containing sugar molecules such as glucose, sucrose, maltose, and hyaluronic acid; and aqueous solutions containing water-soluble polymers such as polyethylene glycol and polyvinyl alcohol. Preferred examples of the physiologically acceptable aqueous solution include an aqueous hyaluronic acid solution containing hyaluronic acid or a salt thereof (sodium hyaluronate or the like).

After the application of the non-lamellar liquid crystal-forming composition as a liquid crystal precursor composition, an aqueous medium is preferably applied onto the non-lamellar liquid crystal-forming composition, but the application manner is not limited to this. An aqueous medium may be applied by a similar application method to that for the non-lamellar liquid crystal-forming composition, for example, by spraying, coating, or injection. After an aqueous medium is applied to a tissue or an organ in this manner, the coating formation is preferably promoted by allowing the applied tissue or organ to stand for a certain period of time (which is not limited but is, for example, 1 to 30 minutes, preferably 5 to 10 minutes).

A typical subject (patient) to whom the adhesion prevention method using the non-lamellar liquid crystal-forming composition of the present invention is applied is a mammal such as a human, livestock, a pet animal, and a laboratory animal. Particularly preferred is a subject who has received or is to receive injury to a tissue (an organ) due to surgery, trauma, diseases, or the like. Surgery includes endoscopic surgery and laparoscopic surgery as well as laparotomy.

The non-lamellar liquid crystal-forming composition of the present invention has a markedly reduced toxicity or no toxicity, and thus the adhesion prevention method pertaining to the present invention is highly safe for a patient to be treated.

The non-lamellar liquid crystal-forming composition pertaining to the present invention may comprise a drug in addition to the above components such as the fatty acid ester and the phospholipid. In the present invention, the drug is any substance (active ingredient) that is contained in the non-lamellar liquid crystal-forming composition and retained in the non-lamellar liquid crystal structure for sustained release (controlled release) and is to be administered to a living body. The drug is not the fatty acid ester itself. The drug may be an organic compound or an inorganic compound. The drug may be a water-soluble drug or a lipid-soluble (lipophilic, water-insoluble, or poorly water-soluble) drug. The drug may be a physiologically active substance. The drug may be, for example, a protein, a peptide, an amino acid, a nucleic acid, or the like but is not limited to them. The drug may be, for example, a gonadotropin-releasing hormone (GnRH) agonist but is not limited to it. The gonadotropin-releasing hormone (GnRH) agonist may be, for example leuprolide or a salt thereof but is not limited to them. The salt of leuprolide may be any pharmaceutically acceptable salt, and examples include, but are not limited to, carboxylates including an acetate (i.e., leuprolide acetate). Leuprolide acetate may be also called leuprorelin acetate or the like. Such a non-lamellar liquid crystal-forming composition may be used for sustained release of a drug.

The present invention also provides a pharmaceutical formulation comprising the non-lamellar liquid crystal-forming composition pertaining to the present invention. The pharmaceutical formulation pertaining to the present invention comprises a non-lamellar liquid crystal-forming composition comprising a fatty acid ester and a phospholipid and having an improved biocompatibility due to the phospholipid. In an embodiment, the pharmaceutical formulation pertaining to the present invention comprises the non-lamellar liquid crystal-forming composition comprising the fatty acid ester, a phospholipid, and a drug. In the present invention, the "pharmaceutical formulation" may be a pharmaceutical composition. The pharmaceutical formulation or the pharmaceutical composition in the present invention may further comprise other substances such as pharmaceutically acceptable additives (for example, carriers, excipients, lubricants, disintegrants, wetting agents, buffers, corrigents, preservatives, colorants, flavoring agents, and propellants) as long as the non-lamellar liquid crystal formability can be maintained.

The pharmaceutical formulation pertaining to the present invention may be formulated in any dosage form and may be, for example, a spray formulation, an aerosol formulation, an injection, or a depot formulation.

The pharmaceutical formulation pertaining to the present invention may be for adhesion prevention of living tissue. The pharmaceutical formulation pertaining to the present invention may be a sustained release formulation further

comprising such a drug as above, such as a depot formulation.

The present invention also provides a method for delivering a drug in a sustained release manner into a living body (into the body) or to live cells or live tissue. The method comprises applying the pharmaceutical formulation pertaining to the present invention comprising a drug or the non-lamellar liquid crystal-forming composition pertaining to the present invention comprising a drug to a living body such as a subject (patient), for example, into the body (specifically, living tissue in the body), onto the body surface, or to living cells or living tissue. When the non-lamellar liquid crystal-forming composition as a liquid crystal precursor composition or a pharmaceutical formulation comprising the composition is used, an aqueous medium may be applied onto the applied non-lamellar liquid crystal-forming composition or the applied pharmaceutical formulation, but the application manner is not limited to this. Alternatively, water in a living body may be used to form liquid crystals. The application to a living body (for example, into the body or onto the body surface), living cells, or living tissue is preferably performed by parenteral administration (such as intravenous, intraarterial, intraperitoneal, intramuscular, subcutaneous, or intracutaneous administration). The method of the invention enables delivery of a drug in a sustained release manner into the body or to living cells or living tissue with high safety.

## Examples

The present invention will next be described more specifically with reference to examples. However, the technical scope of the present invention is not limited to these examples.

[Example 1] Synthesis of amphiphilic compound (fatty acid ester)

(1) Synthesis of propylene glycol monooleate

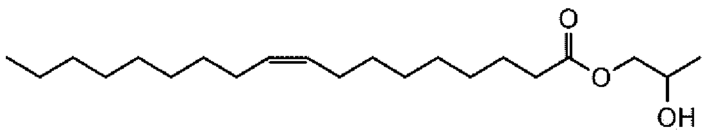

Under a reduced pressure of 8 to 9 kPa, to a solution of 45.6 g (600 mmol) of propylene glycol and 290 mg (2.10 mmol) of potassium carbonate in dry N,N-dimethylformamide (105 mL), 46.6 g of ethyl oleate (150 mmol, olive ethyl oleate NIKKOL EOO, Nikko Chemicals, a typical composition example of main fatty acids in olive oil: 75% of oleic acid, 12% of palmitic acid, 7% of linoleic acid, 3% of stearic acid, and 1% of palmitoleic acid) was added dropwise over 1 hour at 85°C, and the whole was stirred at the same temperature for 2 hours. To the mixture, 83 mg (0.60 mmol) of potassium carbonate was further added, and the whole was stirred at the same temperature for 1 hour. The reaction solution was cooled to 65°C and was neutralized with 283 μL (7.5 mmol) of formic acid, and N,N-dimethylformamide was distilled off under a reduced pressure of 1 to 2 kPa. The resulting solution was diluted with a mixed solvent of ethyl acetate/hexane (1:1, 140 mL), washed with water, a saturated aqueous sodium bicarbonate solution, and saturated brine (twice), and then dried over magnesium sulfate. After filtration, the filtrate was concentrated, and the resulting residue was purified by silica gel column chromatography (mobile phase: hexane/ethyl acetate = 100:0 to 40:60) to give 41.5 g of the title compound (yield 81%) as a low viscous pale yellow transparent liquid. The result of $^1$H-NMR measurement of the resulting compound is as shown below.

$^1$H-NMR spectrum (300 MHz, $CDCl_3$, TMS) δ: 0.88 (m, 3H), 1.2-1.4 (m, 23H), 1.64 (m, 2H), 1.95-2.15 (m, 4H), 2.26-2.38 (m, 2H), 3.55-3.70 (m, 0.7H), 3.9-4.15 (m, 2.0H), 4.99 (m, 0.3H), 5.35 (m, 2H)

Propylene glycol monooleate is also called propylene glycol monooleate (C18:1).

(2) Synthesis of propylene glycol monomyristate

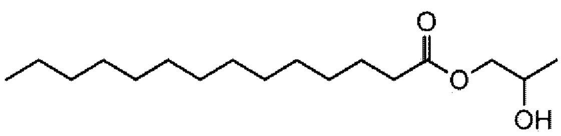

Under a reduced pressure of 8 to 9 kPa, a solution of 2.70 g (10.0 mmol) of isopropyl myristate, 2.43 g (31.9 mmol) of propylene glycol, and 25 mg (0.18 mmol) of potassium carbonate in dry N,N-dimethylformamide (7.0 mL) was stirred at 85°C for 8 hours. The reaction solution was cooled to room temperature and then was neutralized with 19 μL (0.50 mmol) of formic acid. The resulting solution was diluted with a mixed solvent of ethyl acetate/hexane (1:1, 35 mL), washed with water, a saturated aqueous sodium bicarbonate solution, and saturated brine (twice), and then dried over magnesium sulfate. After filtration, the filtrate was concentrated to give 2.80 g of the title compound (yield 98%) as a low viscous colorless transparent liquid. The result of $^1$H-NMR measurement of the resulting compound is as shown below.

$^1$H-NMR spectrum (300 MHz, $CDCl_3$, TMS) δ: 0.88 (t, J = 6.7 Hz, 3H), 1.2-1.4 (m, 23H), 1.63 (m, 2H), 2.26-2.38 (m, 2H), 3.56-3.72 (m, 0.7H), 3.9-4.2 (m, 1.9H), 5.0 (m, 0.4H)

Propylene glycol monomyristate is also called propylene glycol monomyristate (C14:0).

(3) Synthesis of propylene glycol monopalmitate

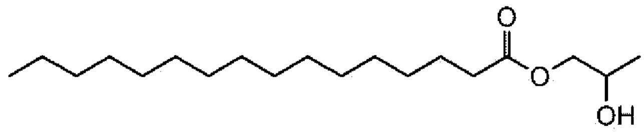

Substantially the same operation as in Example 1(2) was performed except that 2.99 g (10.0 mmol) of isopropyl palmitate was used in place of 2.70 g (10.0 mmol) of isopropyl myristate in Example 1(2), giving 3.12 g of the title compound (yield 99%) as a waxy solid. The result of $^1$H-NMR measurement of the resulting compound is as shown below.

$^1$H-NMR spectrum (300 MHz, $CDCl_3$, TMS) δ: 0.88 (t, J = 6.6 Hz, 3H), 1.2-1.4 (m, 27H), 1.62 (m, 2H), 2.26-2.38 (m, 2H), 3.56-3.72 (m, 0.7H), 3.9-4.2 (m, 1.9H), 5.0 (m, 0.4H)

Propylene glycol monopalmitate is also called propylene glycol monopalmitate (C16:0).

(4) Synthesis of propylene glycol monolinoleate

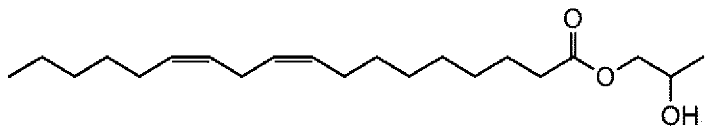

Under a reduced pressure of 8 to 9 kPa, a solution of 3.09 g (10.0 mmol) of ethyl linoleate, 2.43 g (31.9 mmol) of propylene glycol, and 25 mg (0.18 mmol) of potassium carbonate in dry N,N-dimethylformamide (7.0 mL) was stirred at 85°C for 2 hours. The reaction solution was cooled to room temperature and then was neutralized with 19 μL (0.50 mmol) of formic acid. The resulting solution was diluted with a mixed solvent of ethyl acetate/hexane (1:1, 35 mL), washed with water, a saturated aqueous sodium bicarbonate solution, and saturated brine (twice), and then dried over magnesium sulfate. After filtration, the filtrate was concentrated, and the resulting residue was purified by silica gel column chromatography (mobile phase: hexane/ethyl acetate = 100:0 to 40:60) to give 2.45 g of the title compound (yield 72%) as a low viscous colorless transparent liquid. The result of $^1$H-NMR measurement of the resulting compound is as shown below.

$^1$H-NMR spectrum (300 MHz, $CDCl_3$, TMS) δ: 0.89 (m, 3H), 1.2-1.4 (m, 17H), 1.63 (m, 2H), 1.95-2.10 (m, 4H), 2.30-2.38 (m, 2H), 2.77 (m, 2H), 3.55-3.70 (m, 0.7H), 3.89-4.14 (m, 2.0H), 5.0 (m, 0.3H), 5.27-5.44 (m, 4H)

Propylene glycol monolinoleate is also called propylene glycol monolinoleate (C18:2).

(5) Synthesis of propylene glycol monobehenate

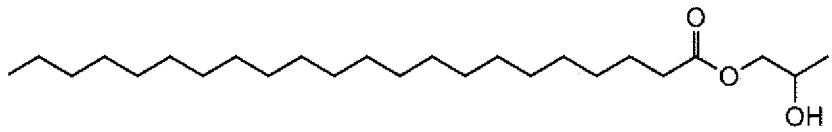

Under a nitrogen atmosphere, to a solution of 3.41 g (10.0 mmol) of behenic acid in dry methylene chloride (30 mL), 1.3 mL (15 mmol) of oxalyl chloride was added dropwise at room temperature. The whole was stirred at the same temperature for 2 hours and was concentrated under reduced pressure. A solution of the resulting residue in dry methylene chloride (20 mL) was added to a solution of 1.14 g (15.0 mmol) of propylene glycol and 1.61 mL (20.0 mmol) of pyridine in dry N,N-dimethylformamide (20 mL) under a nitrogen atmosphere at 0°C, and the mixture was immediately stirred at room temperature overnight. The resulting reaction solution was diluted with a mixed solvent of ethyl acetate/hexane (1:1, 60 mL), washed with water, 1 M hydrochloric acid, a saturated aqueous sodium bicarbonate solution, and saturated brine, and then dried over magnesium sulfate. After filtration, the filtrate was concentrated to give 3.82 g of the title compound (yield 96%) as a white powder. The result of $^1$H-NMR measurement of the resulting compound is as shown below.

$^1$H-NMR spectrum (300 MHz, $CDCl_3$, TMS) δ: 0.88 (t, J = 6.6 Hz, 3H), 1.2-1.4 (m, 39H), 1.63 (m, 2H), 2.26-2.38 (m, 2H), 3.58-3.72 (m, 0.4H), 3.89-4.19 (m, 2.4H), 5.0 (m, 0.2H)

Propylene glycol monobehenate is also called propylene glycol monobehenate (C22:0).

(6) Synthesis of ethylene glycol monooleate

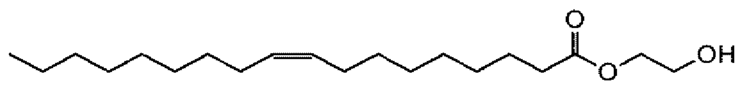

Substantially the same operation as in Example 1(4) was performed except that 3.11 g (10.0 mmol) of ethyl oleate and 1.98 g (31.9 mmol) of ethylene glycol were used in place of 3.09 g (10.0 mmol) of ethyl linoleate and 2.43 g (31.9 mmol) of propylene glycol in Example 1(4), giving 2.88 g of the title compound (yield 88%) as a colorless transparent liquid. The result of $^1$H-NMR measurement of the resulting compound is as shown below.

$^1$H-NMR spectrum (300 MHz, $CDCl_3$, TMS) δ: 0.88 (m, 3H), 1.2-1.4 (m, 20H), 1.63 (m, 2H), 1.95-2.1 (m, 4H), 2.35 (t, J = 7.6 Hz, 2H), 3.83 (m, 2H), 4.21 (m, 2H), 5.34 (m, 2H)

Ethylene glycol monooleate is also called ethylene glycol monooleate (C18:1).

(7) Synthesis of 1,3-butylene glycol monooleate

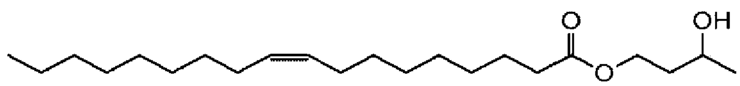

Substantially the same operation as in Example 1(4) was performed except that 3.11 g (10.0 mmol) of ethyl oleate and 2.87 g (31.9 mmol) of 1,3-butylene glycol were used in place of 3.09 g (10.0 mmol) of ethyl linoleate and 2.43 g (31.9 mmol) of propylene glycol in Example 1(4), giving 2.90 g of the title compound (yield 82%) as a low viscous colorless transparent liquid. The result of $^1$H-NMR measurement of the resulting compound is as shown below.

$^1$H-NMR spectrum (300 MHz, $CDCl_3$, TMS) δ: 0.88 (m, 3H), 1.2-1.4 (m, 23H), 1.62 (m, 2H), 1.6-1.9 (m, 2H), 1.95-2.1 (m, 4H), 2.26-2.34 (m, 2H), 3.5-3.7 (m, 0.5H), 3.86 (m, 0.75H), 4.12 (m, 0.75H), 4.35 (ddd, J = 5.3, 8.3, 13.6 Hz, 0.75H), 5.12 (m, 0.25H), 5.34 (m, 2H)

1,3-Butylene glycol monooleate is also called butylene glycol monooleate (C18:1).

(8) Synthesis of 3-methyl-1,3-butanediol monooleate

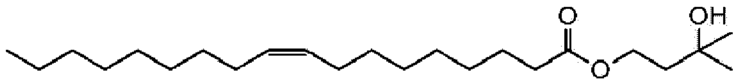

Substantially the same operation as in Example 1(4) was performed except that 3.11 g (10.0 mmol) of ethyl oleate and 3.32 g (31.9 mmol) of 3-methyl-1,3-butanediol were used in place of 3.09 g (10.0 mmol) of ethyl linoleate and 2.43 g (31.9 mmol) of propylene glycol in Example 1(4), giving 2.31 g of the title compound (yield 63%) as a low viscous colorless transparent liquid. The result of $^1$H-NMR measurement of the resulting compound is as shown below.

$^1$H-NMR spectrum (300 MHz, $CDCl_3$, TMS) δ: 0.88 (m, 3H), 1.2-1.4 (m, 26H), 1.61 (m, 2H), 1.84 (t, J = 6.9 Hz, 2H), 1.95-2.1 (m, 4H), 2.30 (t, J = 7.5 Hz, 2H), 4.25 (t, J = 6.9 Hz, 2H), 5.34 (m, 2H)

3-Methyl-1,3-butanediol monooleate is also called isoprene glycol monooleate (C18:1).

(9) Synthesis of diethylene glycol monooleate

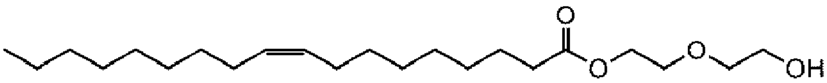

Substantially the same operation as in Example 1(4) was performed (0.08 equivalents of potassium carbonate was added) except that 3.11 g (10.0 mmol) of ethyl oleate and 3.39 g (31.9 mmol) of diethylene glycol were used in place of 3.09 g (10.0 mmol) of ethyl linoleate and 2.43 g (31.9 mmol) of propylene glycol in Example 1(4), giving 2.57 g of the title compound (yield 69%) as a low viscous pale yellow transparent liquid. The result of $^1$H-NMR measurement of the resulting compound is as shown below.

$^1$H-NMR spectrum (300 MHz, $CDCl_3$, TMS) δ: 0.88 (m, 3H), 1.2-1.4 (m, 20H), 1.63 (m, 2H), 1.95-2.1 (m, 4H), 2.28-2.38 (m, 2H), 3.61 (m, 2H), 3.71 (m, 2H), 3.74 (m, 2H), 4.25 (m, 2H), 5.34 (m, 2H)

Diethylene glycol monooleate is also called diethylene glycol monooleate (C18:1).

[Example 2] Preparation of precursor formulation, gel formation test, and, liquid crystal structure analysis

In accordance with the combination ratios shown in Table 1 and Table 2, an amphiphilic compound synthesized in Example 1 or propylene glycol monocaprylate (C8:0) (propylene glycol monocaprylate) (NIKKOL SEFSOL-218, Nikko Chemicals) as the amphiphilic compound having a hydrophilic group with one hydroxyl group (fatty acid ester), soybean phosphatidylcholine (soybean lecithin, LIPOID S100, Lipoid GmbH, SPC is used as the abbreviation hereinafter and in Tables), egg yolk phosphatidylcholine (purified egg yolk lecithin, PL-100M, Kewpie Corp., EPC is used as the abbreviation hereinafter and in Tables), or dioleyl phosphatidylethanolamine (COATSOME ME-8181, NOF Corp., DOPE is used as the abbreviation hereinafter and in Tables) as the phospholipid, sesame oil (triglyceride, sesame oil under the Japanese Pharmacopoeia, Kaneda Co., Ltd.) as the oil, and EtOH (ethanol) were mixed. The resulting mixture was dissolved in a water bath at 40°C or less, and precursor formulations Nos. 1 to 23 shown in Tables 1 and 2 were prepared.

The precursor formulations Nos. 1 to 23 were subjected to gel formation test. A portion (about 100 to 300 mg) of each precursor formulation was added to an excess amount (about 0.5 to 2 mL) of water for injection in a vial, and the whole was mixed at room temperature (25°C) with a spatula and/or a vortex mixer. As a result, all the precursor formulations Nos. 1 to 23 gave gel compositions that were colorless transparent to cloudy white in appearance and were separated in the excess water for injection (aqueous medium).

The gel compositions formed from the precursor formulations Nos. 1 to 23 were each directly embedded in a pinhole slit and subjected to small-angle X-ray scattering diffractometry with a small-angle x-ray scattering (SAXS) apparatus (manufactured by Rigaku Corp., Nano-Viewer) to analyze the non-lamellar liquid crystal structure.

The obtained liquid crystal phases and scattering vector values q1 [$nm^{-1}$] of peaks located at the smallest angle are shown in Tables 1 and 2. HII means reverse hexagonal liquid crystals, Pn3m means reverse cubic liquid crystals belonging to the crystallographic space group Pn3m, Im3m means reverse cubic liquid crystals belonging to the crystallographic space group Im3m, and Fd3m means reverse cubic liquid crystals belonging to the crystallographic space group Fd3m. There were some gel compositions having two types of different liquid crystal phases.

Of the used amphiphilic compounds having a hydrophilic group with one hydroxyl group, diethylene glycol monooleate formed a gel composition when mixed with water alone (liquid crystal phase: HII, q1: 1.35 $nm^{-1}$). The amphiphilic compounds other than diethylene glycol monooleate failed to form a gel composition when mixed with water alone.

As shown in Tables 1 and 2, the precursor formulations were suggested to form non-lamellar liquid crystals in

a wide range of ratios, amphiphilic compound having a hydrophilic group with one hydroxyl group:phospholipid (weight ratios). The precursor formulations were able to form non-lamellar liquid crystals in a wide range of ratios, the amphiphilic compound + phospholipid (the total amount of the amphiphilic compound and the phospholipid):oil (weight ratios). It was revealed that various combinations of the amphiphilic compound, the phospholipid, and the oil provided a wide variety of liquid crystal structures such as reverse cubic liquid crystals belonging to the crystallographic space group Pn3m, Im3m, or Fd3m and reverse hexagonal liquid crystals and gave a very wide range of q1 values, which are attributed to the lattice constant.

[Table 1]

Table 1

| Formulation No. | Amphiphilic compound | Composition | | | | Liquid crystal formation with water | |
|---|---|---|---|---|---|---|---|
| | | Lipid and oil | | | EtOH [%] | SAXS | |
| | | Amphiphilic compound + SPC + oil [%] | Amphiphilic compound: SPC [weight ratio] | (Amphiphilic compound + SPC): sesame oil [weight ratio] | | Liquid crystal phase | q1 [$nm^{-1}$] |
| 1 | Propylene glycol monooleate (C18:1) | 92.6 | 70:30 | 100:0 | 7.4 | Fd3m | 0.67 |
| 2 | | 92.6 | 60:40 | 100:0 | 7.4 | HII | 1.24 |
| 3 | | 100 | 60:40 | 100:0 | 0 | HII | 1.24 |
| 4 | | 92.6 | 50:50 | 100:0 | 7.4 | HII | 1.11 |
| 5 | | 92.6 | 40:60 | 100:0 | 7.4 | Pn3m+HII | 0.57+1.02 |
| 6 | | 92.6 | 30:70 | 100:0 | 7.4 | Im3m | 0.43 |
| 7 | | 92.6 | 50:50 | 90:10 | 7.4 | HII | 1.05 |
| 8 | | 92.6 | 40:60 | 95:5 | 7.4 | HII | 0.93 |
| 9 | | 92.6 | 40:60 | 70:30 | 7.4 | HII | 0.76 |
| 10 | | 92.6 | 40:60 | 60:40 | 7.4 | HII | 0.69 |
| 11 | | 92.6 | 40:60 | 50:50 | 7.4 | Im3m | 0.43 |
| 12 | | 92.6 | 30:70 | 85:15 | 7.4 | Pn3m+HII | 0.45+0.68 |
| 13 | Propylene glycol monocaprylate (C8:0) | 92.6 | 50:50 | 100:0 | 7.4 | HII | 1.08 |
| 14 | Propylene glycol monomyristate (C14:0) | 92.6 | 50:50 | 100:0 | 7.4 | HII | 1.03 |
| 15 | Propylene glycol monopalmitate (C16:0) | 92.6 | 50:50 | 100:0 | 7.4 | Im3m | 0.45 |
| 16 | Propylene glycol monolinoleate (C18:2) | 92.6 | 50:50 | 100:0 | 7.4 | HII | 1.15 |

(continued)

| Formulation No. | Amphiphilic compound | Composition | | | | Liquid crystal formation with water | |
|---|---|---|---|---|---|---|---|
| | | Lipid and oil | | | EtOH [%] | SAXS | |
| | | Amphiphilic compound + SPC + oil [%] | Amphiphilic compound: SPC [weight ratio] | (Amphiphilic compound + SPC): sesame oil [weight ratio] | | Liquid crystal phase | q1 [$nm^{-1}$] |
| 17 | Ethylene glycol monooleate (C18:1) | 92.6 | 50:50 | 100:0 | 7.4 | HII | 1.05 |
| 18 | Butylene glycol monooleate (C18:1) | 92.6 | 50:50 | 100:0 | 7.4 | HII | 1.08 |
| 19 | Isoprene glycol monooleate (C18:1) | 92.6 | 50:50 | 100:0 | 7.4 | HII | 1.07 |
| 20 | Diethylene glycol monooleate (C18:1) | 92.6 | 80:20 | 100:0 | 7.4 | Pn3m | 0.95 |
| 21 | Diethylene glycol monooleate (C18:1) | 92.6 | 60:40 | 100:0 | 7.4 | Pn3m | 0.68 |

[Table 2]

Table 2

| Formulation No. | Amphiphilic compound | Composition | | | | Liquid crystal formation with water | |
|---|---|---|---|---|---|---|---|
| | | Lipid and oil | | | EtOH [%] | SAXS | |
| | | Amphiphilic compound + phospholipid + oil [%] | Amphiphilic compound: phospholipid [weight ratio]* | (Amphiphilic compound + phospholipid): sesame oil [weight ratio] | | Liquid crystal phase | q1 [$nm^{-1}$] |
| 22 | Propylene glycol monooleate (C18:1) | 92.6 | 50:50 EPC | 100:0 | 7.4 | HII | 1.11 |
| 23 | | 92.6 | 50:50 DOPE | 100:0 | 7.4 | HII | 1.41 |
| * The used phospholipids are given after the weight ratios. | | | | | | | |

[Example 3] Preparation of emulsion (dispersion) and analysis of liquid crystal structure/particle size distribution

In accordance with the combination ratios shown in Table 3, propylene glycol monooleate (C18:1) (synthesized in Example 1) as the amphiphilic compound having a hydrophilic group with one hydroxyl group, SPC as the phospholipid, sesame oil as the oil, and EtOH were mixed to give an oil solution. Separately, Pluronic F127 (Unilube (registered trademark) 70DP-950B, NOF Corp.) as a surfactant and water for injection (Otsuka distilled water) were mixed to give an aqueous Pluronic solution. The oil solution and the aqueous Pluronic solution prepared as above were each completely dissolved in a water bath at 40°C or less and then were mixed together at room temperature. The mixture was stirred with a spatula or a stirrer tip to give a suspension. The suspension was dispersed with a high-pressure homogenizer (Star Burst Minimo, manufactured by Sugino Machine) to give a white emulsion containing fine particles (formulations Nos. 24 and 25). These emulsions were each prepared in an amount of 8 g.

The emulsions as the formulations Nos. 24 and 25 prepared as above were subjected to structure analysis by small-angle X-ray scattering (SAXS) with a NANO Viewer nano-scale X-ray structure analyzer (manufactured by Rigaku). Each emulsion was introduced to a capillary under atmospheric pressure and was measured in the apparatus under a reduced pressure (the sample itself was under atmospheric pressure). In the scattering intensity distribution obtained from the emulsion as the formulation No. 24, at least three scattering peaks were observed. The peak ratio showed a ratio of 1:$\sqrt{3}$:2 (q1: 1.22 $nm^{-1}$) specific to reverse hexagonal liquid crystals, and this revealed that the emulsion was a liquid crystal emulsion (hexasome) in which fine particles of reverse hexagonal liquid crystals were dispersed in an aqueous phase. In the scattering intensity distribution obtained from the emulsion as the formulation No. 25, a broad scattering peak was observed, and this suggested that the emulsion was a liquid crystal emulsion in which fine particles in the sponge phase (L3 phase) were dispersed in an aqueous phase.

The emulsions as the formulations Nos. 24 and 25 were further subjected to particle size distribution measurement by dynamic light scattering with a Zetasizer Nano-ZS (manufactured by Malvern). The measurement sample was prepared by diluting each emulsion immediately after the preparation (within four days at room temperature) 200-fold with distilled water. Table 3 shows the average particle size (nm) (Z-Average) and PDI (polydispersity index) obtained from each measurement sample. Each emulsion was stable in appearance after three months at room temperature.

In a wide range of ratios of the amphiphilic compound:the phospholipid and ratios of the amphiphilic compound + phospholipid (the total amount of the amphiphilic compound and the phospholipid):oil shown in Table 3, the liquid crystal emulsion containing non-lamellar liquid crystals stable over time was able to be prepared.

[Table 3]

Table 3

| Formulation No. | Amphiphilic compound | Composition | | | | | | Particle size distribution | |
|---|---|---|---|---|---|---|---|---|---|
| | | Lipid and oil | | | EtOH [%] | Pluronic F127 [%] | Water for injection [%] | Average particle size (nm) | PDI |
| | | Amphiphilic compound + SPC + oil [%] | Amphiphilic compound:SPC [weight ratio] | (Amphiphilic compound + SPC):sesame oil [weight ratio] | | | | | |
| 24 | Propylene glycol monooleate (C18: 1) | 25 | 60:40 | 100:0 | 2 | 5 | 68 | 131 | 0.13 |
| 25 | | 25 | 40:60 | 70:30 | 2 | 5 | 68 | 113 | 0.16 |

[Example 4] Preparation of precursor formulation of amphiphilic compound having hydrophilic group with different number of hydroxyl groups, property identification, gel formation test, and viscoelastic test

Characteristics of precursor formulations each comprising an amphiphilic compound having a hydrophilic group with 1 to 3 hydroxyl groups and a phospholipid were compared.

Propylene glycol monooleate (C18:1) (synthesized in Example 1) was used as the amphiphilic compound having a hydrophilic group with one hydroxyl group, glyceryl monooleate (Rikemal XO-100, NOF Corp.) was used as the amphiphilic compound having a hydrophilic group with two hydroxyl groups, and purified sorbitan monooleate (prepared by removing low-polarity components such as oleic acid and sorbitan dioleate from sorbitan monooleate NIKKOL SO-10 V (Nikko Chemicals) by silica gel column purification) was used as the amphiphilic compound having a hydrophilic group with three hydroxyl groups. Propylene glycol monooleate (C18:1) is a low viscous pale yellow transparent liquid, whereas glyceryl monooleate is a waxy solid and purified sorbitan monooleate is a viscous fluid.

In accordance with the combination ratios shown in Table 4, materials were mixed and dissolved in a similar manner to that in Example 2 to give precursor formulations Nos. 26 to 31 each in an amount of 2.2 g.

The precursor formulations Nos. 26 and 27 containing propylene glycol monooleate (C18:1) were able to be prepared in a shorter period of time than the precursor formulations Nos. 28 and 30 having an amphiphilic compound:phospholipid ratio of 60:40 and containing different amphiphilic compounds or the precursor formulations Nos. 29 and 31 having an amphiphilic compound:phospholipid ratio of 40:60 and containing different amphiphilic compounds. The time for preparation was within 1 hour for the precursor formulations Nos. 26 and 27 and was about 2 hours for the precursor formulations Nos. 28 to 31. The precursor formulations Nos. 28 to 31 containing glyceryl monooleate or purified sorbitan monooleate were able to be homogeneously dissolved and prepared only when an organic solvent (e.g., EtOH) was used. In contrast, the precursor formulation No. 26 containing propylene glycol monooleate (C18:1) was able to be homogeneously dissolved and prepared without any organic solvent (see the formulation No. 3 in Example 2).

As for the precursor formulation No. 28 containing glyceryl monooleate and having an amphiphilic compound:phospholipid ratio of 60:40, glyceryl monooleate started to precipitate around 20°C, and the formulation was solidified under refrigeration.

The precursor formulations Nos. 26 to 31 were subjected to gel formation test in a similar manner to that in Example 2. As a result, the precursor formulations Nos. 26 to 28, 30, and 31 gave gel compositions that were colorless transparent to cloudy white in appearance and were separated in the excess water for injection (aqueous medium) (a small amount of EtOH less affected the non-lamellar liquid crystal structure, and the precursor formulations Nos. 2 and 3 and No. 26 and the precursor formulations Nos. 5 and 27 each gave substantially the same non-lamellar liquid crystal structure). In contrast, the precursor formulation No. 29 containing glyceryl monooleate and having an amphiphilic compound:phospholipid ratio of 40:60 failed to give a gel composition, and the whole solution in a vial gave an emulsion.

The precursor formulations Nos. 26 to 31 were subjected to shear viscosity measurement by using a viscosity/viscoelasticity measuring instrument (MCR302, Anton Paar; a cone plate of ϕ50, a cone angle of 1°, a temperature of 23 to 25°C). Table 4 shows viscosities [Pa·s] at a shear velocity of 1 (1/s) or 100 (1/s).

In each case of comparing the precursor formulations Nos. 26, 28, and 30 having an amphiphilic compound:phospholipid ratio of 60:40 and comparing the precursor formulations Nos. 27, 29, and 31 having an amphiphilic compound:phospholipid ratio of 40:60, the precursor formulation containing propylene glycol monooleate (C18:1) (each of the precursor formulations Nos. 26 and 27) had the lowest viscosity at a shear velocity of 1 (1/s) and 100 (1/s). Of them, even the precursor formulation No. 27 having an amphiphilic compound:phospholipid ratio of 40:60 and having a higher viscosity had a lower viscosity than the precursor formulations Nos. 28 to 31 containing other lipids.

The above results revealed that the precursor formulation comprising an amphiphilic compound having a hydrophilic group with one hydroxyl group and a phospholipid was easily prepared and stable, had a high non-lamellar liquid crystal structure formability, and had a markedly low viscosity, in a wide range of ratios of the amphiphilic compound:the phospholipid, as compared with the precursor formulations comprising an amphiphilic compound having a hydrophilic group with two or three hydroxyl groups and a phospholipid (the fatty acid in each amphiphilic compound was oleic acid). When a composition having a low viscosity is used, for example, as an injection, a thinner needle can be used advantageously.

[Table 4]

Table 4

| Formulation No. | Amphiphilic compound | Composition | | | Viscosity [Pa·s] | |
|---|---|---|---|---|---|---|
| | | Amphiphilic compound + SPC [%] | Amphiphilic compound: SPC [weight ratio] | EtOH [%] | Shear velocity | |
| | | | | | 1 (1/s) | 100 (1/s) |
| 26 | Propylene glycol monooleate (C18:1) | 90 | 60:40 | 10 | 0.88 | 0.10 |
| 27 | | 90 | 40:60 | 10 | 5.25 | 0.40 |
| 28 | Glyceryl monooleate (C18: 1) | 90 | 60:40 | 10 | 830 | 1.32 |
| 29 | | 90 | 40:60 | 10 | 564 | 2.32 |
| 30 | Purified sorbitan monooleate (C18:1) | 90 | 60:40 | 10 | 5.68 | 0.62 |
| 31 | | 90 | 40:60 | 10 | 9.91 | 1.53 |

[Example 5] Preparation of precursor formulation containing leuprolide acetate and in vitro release test

In accordance with the combination ratios shown in Table 5, propylene glycol monooleate (C18:1) as the amphiphilic compound having a hydrophilic group with one hydroxyl group, SPC as the phospholipid, sesame oil as the oil, and EtOH were mixed, and the mixture was dissolved in a water bath at 40°C or less. Subsequently, a solution of leuprolide acetate (L0249, Tokyo Chemical Industry Co., Ltd., LA is used as the abbreviation hereinafter and in Table) in dimethyl sulfoxide (DMSO) was added and homogeneously dissolved to give precursor formulations Nos. 32 to 35 containing leuprolide acetate.

The precursor formulations Nos. 32, 33, and 35 (100 mg) containing leuprolide acetate were subjected to in vitro release test. In one vial (a capacity of 25 mL) containing 20 mL of pH 7.4 PBS solution containing 0.02% P80 (polyoxyethylene sorbitan monooleate (20E.O.), one dialysis tube having an upper part connected to a floating rack was placed such that a precursor formulation placed in the tube was sufficiently immersed in the PBS solution, and the whole was allowed to stand at room temperature (25°C). Next, 500 μL of the PBS solution in the vial was collected after 0, 1, 3, 6, 12, 24, 48, 72, 120, and 168 hours from the start of the test over 7 days. It was visually observed that each of the precursor formulations No. 32, 33, and 35 added to the above PBS solution formed a gel composition.

Leuprolide acetate in a sample collected from the PBS solution was quantified by LC/MS/MS analysis using a previously prepared calibration curve. The analysis conditions were as described below.

· Analytical column; Shodex ODP2HP-2B, 2.0 mmI.D. × 50 mm (Showa Denko K.K.)

- Mobile phase; water (containing 0.1% formic acid):acetonitrile = 70:30
- Flow rate; 0.1 mL/min, column temperature; 40°C, injection volume; 10 μL
- Precursor ion; 605.3 m/z, product ion; 249.0 m/z

FIG. 1 shows in vitro release data of the precursor formulations Nos. 32, 33, and 35 containing leuprolide acetate. In FIG. 1, the horizontal axis indicates time [days], and the vertical axis indicates the release rate [%] (average from n = 3) of leuprolide acetate into the PBS solution at each time point to the amount of leuprolide acetate contained in the precursor formulation at the start of the test. It was revealed that the precursor formulations Nos. 32, 33, and 35 each showed no initial burst release and achieved sustained release of leuprolide acetate. Of them, the precursor formulations Nos. 33 and 35 had a high sustained release rate, whereas the precursor formulation No. 32 had a low sustained release rate.

The weight ratios of lipid and oil in the precursor formulations Nos. 32, 33, and 35 were the same as those in the precursor formulations Nos. 8, 10, and 12, respectively, and gave substantially the same non-lamellar liquid crystal structures by addition to an aqueous medium. The liquid crystal phases and the unit lattices a [nm] of the gel compositions prepared from the precursor formulations Nos. 8, 10, and 12 with water were HII and 7.8 nm, HII and 11 nm, and Pn3m + HII and 20 nm + 11 nm, respectively (from Table 1, the unit lattice a is calculated from a liquid crystal structure and peak scattering vector values such as q1). It is typically thought that a more hydrophilic liquid crystal phase provides a higher sustained release rate (Pn3m > HII > Fd3m) and a larger unit lattice a provides a higher sustained release rate. The resulting sustained release rates were in accordance with the rule.

[Table 5]

Table 5

| Formulation No. | Amphiphilic compound | Composition | | | | | |
|---|---|---|---|---|---|---|---|
| | | Drug solution | | Lipid and oil | | | EtOH [%] |
| | | LA [%] | DMSO [%] | Amphiphilic compound + SPC + oil [%] | Amphiphilic compound: SPC [weight ratio] | (Amphiphilic compound + SPC): sesame oil [weight ratio] | |
| 32 | Propylene glycol monooleate (C18:1) | 3.75 | 5 | 84.25 | 40:60 | 95:5 | 7 |
| 33 | | 3.75 | 5 | 84.25 | 40:60 | 60:40 | 7 |
| 34 | | 3.75 | 5 | 84.25 | 60:40 | 100:0 | 7 |
| 35 | | 3.75 | 5 | 84.25 | 30:70 | 85:15 | 7 |

[Example 6] In vivo drug pharmacokinetic test of precursor formulation containing leuprolide acetate

Eight-week-old male Wistar rats with cannulation of jugular vein in advance underwent general anesthesia with a mixture of three anesthetic agents (medetomidine hydrochloride + midazolam + butorphanol tartrate). The backs were shaved, and then 100 mg of each of the precursor formulations Nos. 34 and 35 (prepared in Example 5) containing leuprolide acetate was subcutaneously administered to the right back using a syringe (Terumo Syringe 1 mL) with a 23G needle.

Next, 100 μL of blood was collected through the cannulation tube after 0, 1, 3, and 6 hours and 1, 3, 5, 7, and 10 days from the start of the test. Each blood sample was centrifuged (15,000 rpm, 5 minutes, 4°C) to give plasma. To 50 μL of each plasma, 50 μL of acetonitrile was added. The whole was stirred with a vortex mixer and then was further centrifuged (15,000 rpm, 5 minutes, 4°C). The resulting supernatant was used as a measurement sample.

Leuprolide acetate in the measurement sample was quantified by LC/MS/MS analysis in a similar manner to that in Example 5.

FIG. 2 shows in vitro pharmacokinetic data of the precursor formulations Nos. 34 and 35 containing leuprolide acetate. In FIG. 2, the horizontal axis indicates time [days], and the vertical axis indicates the blood concentration of leuprolide acetate [ng/mL] (average from n = 5). From FIG. 2, the AUC (area under the blood concentration-time curve) [ng·h/mL] was calculated and was 2,170 for the precursor formulation No. 34 and 2,152 for the precursor formulation No. 35.

The blood concentration of leuprolide acetate disappeared in 7 days for the precursor formulation No. 34 and disappeared within 3 days for the precursor formulation No. 35. The precursor formulations Nos. 34 and 35 had the same weight ratios of lipid and oil as the precursor formulations Nos. 3 and 12, respectively, and gave substantially the same non-lamellar liquid crystal structures by addition to an aqueous medium. The liquid crystal phases and the unit lattices a [nm] of the gel compositions obtained from the precursor formulations Nos. 3 and 12 with water were HII and 5.9 nm and Pn3m + HII and 20 nm + 11 nm, respectively (from Table 1, the unit lattice a is calculated from a liquid crystal structure and peak scattering vector values such as q1). As with the in vitro release test in Example 5, the resulting sustained release rates were in accordance with the rule that a larger unit lattice a provides a higher sustained release rate.

After 28 days of the administration, the rat to which the precursor formulation No. 35 had been administered was euthanized by bleeding under inhalation anesthesia with sevoflurane (Mylan Seiyaku Ltd.), and then the formulation administration site including the skin and subcutaneous tissue was extracted. The subcutaneous fat was roughly removed, and then the formulation administered site was macroscopically observed.

As shown in FIG. 3, which is a photograph of the formulation administration site, the formulation was not left in the formulation administration site, and the inflammation derived from the formulation was not macroscopically observed in the peripheral tissue.

[Example 7] Synthesis of amphiphilic compound (fatty acid ester)

Substantially the same operation as in Example 1(4) was performed (when isosorbide was used, 0.3 equivalents of potassium carbonate was used and the reaction was performed at 95°C) except that 2.87 g (31.9 mmol) of 1,3-butylene

glycol, 3.32 g (31.9 mmol) of 3-methyl-1,3-butanediol, 3.39 g (31.9 mmol) of diethylene glycol, or 4.66 g of isosorbide (31.9 mmol, Sanko Chemical Industry Co., Ltd., a purity of 98.0% or more, a water content of 1.0% or less) was used in place of 2.43 g (31.9 mmol) of propylene glycol in Example 1(4) or except that 3.11 g (10.0 mmol) of ethyl oleate and 4.66 g (31.9 mmol) of isosorbide were used in place of 3.09 g (10.0 mmol) of ethyl linoleate and 2.43 g (31.9 mmol) of propylene glycol in Example 1(4), giving 1,3-butylene glycol monolinoleate, 3-methyl-1,3-butanediol monolinoleate, diethylene glycol monolinoleate, isosorbide monolinoleate, or isosorbide monooleate, as a colorless to pale yellow transparent liquid. The amount, the yield, and the $^1$H-NMR measurement data of each compound were as shown below.

(1) 1,3-Butylene glycol monolinoleate

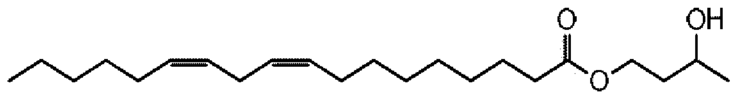

Amount 2.67 g (yield 76%)
$^1$H-NMR spectrum (300 MHz, $CDCl_3$, TMS) $\delta$: 0.89 (m, 3H), 1.2-1.4 (m, 17H), 1.62 (m, 2H), 1.6-1.9 (m, 2H), 1.95-2.1 (m, 4H), 2.30-2.37 (m, 2H), 2.77 (m, 2H), 3.5-3.7 (m, 0.5H), 3.85 (m, 0.75H), 4.12 (m, 0.75H), 4.35 (ddd, J = 5.3, 8.3, 13.6 Hz, 0.75H), 5.13 (m, 0.25H), 5.26-5.44 (m, 4H)

(2) 3-Methyl-1,3-butanediol monolinoleate

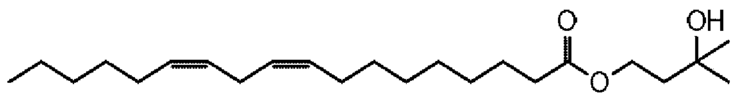

Amount 2.41 g (yield 66%)
$^1$H-NMR spectrum (300 MHz, $CDCl_3$, TMS) $\delta$: 0.89 (m, 3H), 1.2-1.4 (m, 20H), 1.62 (m, 2H), 1.84 (t, J = 6.9 Hz, 2H), 1.95-2.1 (m, 4H), 2.30 (t, J = 7.5 Hz, 2H), 2.77 (m, 2H), 4.25 (t, J = 6.9 Hz, 2H), 5.27-5.43 (m, 4H)

(3) Diethylene glycol monolinoleate

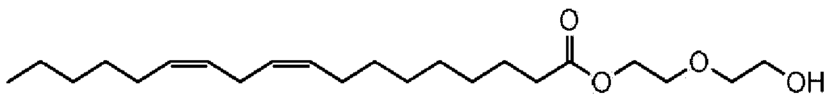

Amount 2.48 g (yield 67%)
$^1$H-NMR spectrum (300 MHz, $CDCl_3$, TMS) $\delta$: 0.89 (m, 3H), 1.2-1.4 (m, 14H), 1.63 (m, 2H), 1.95-2.1 (m, 4H), 2.34 (t, J = 7.6 Hz, 2H), 2.77 (m, 2H), 3.61 (m, 2H), 3.70 (m, 2H), 3.74 (m, 2H), 4.25 (m, 2H), 5.27-5.44 (m, 4H)

(4) Isosorbide monolinoleate

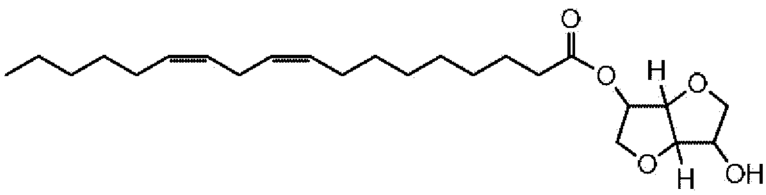

Amount 2.31 g (yield 56%)

$^1$H-NMR spectrum (300 MHz, $CDCl_3$, TMS) δ: 0.89 (m, 3H), 1.2-1.4 (m, 14H), 1.64 (m, 2H), 1.95-2.1 (m, 4H), 2.3-2.4 (m, 2H), 2.77 (m, 2H), 3.55 (dd, J = 6.0, 9.5 Hz, 0.6H), 3.75 (ddd, J = 2.1, 5.2, 9.8 Hz, 0.4H), 3.8-3.95 (m, 1.8 H), 3.99 (m, 1.2H), 4.2-4.35 (m, 1H), 4.38 (d, J = 4.6 Hz, 0.4H), 4.44 (d, J = 4.3 Hz, 0.6H), 4.60 (t, J = 4.9 Hz, 0.6H), 4.83 (t, J = 5.0 Hz, 0.4H), 5.12 (m, 0.4H), 5.21 (s, 0.6H), 5.26-5.45 (m, 4H)

(5) Isosorbide monooleate

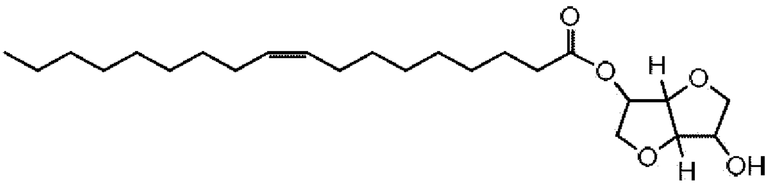

Amount 2.38 g (yield 58%)

$^1$H-NMR spectrum (300 MHz, $CDCl_3$, TMS) δ: 0.88 (m, 3H), 1.2-1.4 (m, 20H), 1.64 (m, 2H), 1.95-2.15 (m, 4H), 2.25-2.4 (m, 2H), 3.54 (dd, J = 6.0, 9.5 Hz, 0.6H), 3.74 (ddd, J = 2.1, 5.2, 9.8 Hz, 0.4H), 3.8-3.95 (m, 1.8H), 3.99 (m, 1.2H), 4.2-4.35 (m, 1H), 4.37 (d, J = 4.6 Hz, 0.4H), 4.44 (d, J = 4.3 Hz, 0.6H), 4.60 (t, J = 4.9 Hz, 0.6H), 4.82 (t, J = 5.0 Hz, 0.4H), 5.12 (m, 0.4H), 5.20 (s, 0.6H), 5.34 (m, 2H)

[Example 8] Preparation of precursor formulation, gel formation test, and liquid crystal structure analysis

In accordance with the combination ratios shown in Table 6, an amphiphilic compound synthesized in Example 7, SPC (LIPOID S100, Lipoid GmbH) as the phospholipid, and EtOH (ethanol) were mixed. The resulting mixture was dissolved in a water bath at 40°C or less, and precursor formulations Nos. 36 to 40 shown in Table 6 were prepared.

1,3-Butylene glycol monolinoleate, 3-methyl-1,3-butanediol monolinoleate, and isosorbide monolinoleate failed to form a gel composition when mixed with water alone.

The precursor formulations Nos. 36 to 40 were subjected to gel formation test. A portion (about 100 to 300 mg) of each precursor formulation was added to an excess amount (about 0.5 to 2 mL) of water for injection in a vial, and the whole was mixed at room temperature (25°C) with a spatula and/or a vortex mixer. As a result, all the precursor formulations Nos. 36 to 40 gave gel compositions that were colorless transparent to cloudy white in appearance and were separated in the excess water for injection (aqueous medium), and these compositions were thought to show non-lamellar liquid crystal structures of reverse hexagonal liquid crystals or cubic liquid crystals.

[Table 6]

Table 6

| Formulation No. | Amphiphilic compound | Composition | | |
|---|---|---|---|---|
| | | Amphiphilic compound + SPC [%] | Amphiphilic compound: SPC [weight ratio] | EtOH [%] |
| 36 | 1,3-Butylene glycol monolinoleate | 92.6 | 50:50 | 7.4 |

(continued)

| Formulation No. | Amphiphilic compound | Composition | | |
|---|---|---|---|---|
| | | Amphiphilic compound + SPC [%] | Amphiphilic compound: SPC [weight ratio] | EtOH [%] |
| 37 | 3-Methyl-1,3-butanediol monolinoleate | 92.6 | 50:50 | 7.4 |
| 38 | Diethylene glycol monolinoleate | 92.6 | 50:50 | 7.4 |
| 39 | Isosorbide monolinoleate | 92.6 | 50:50 | 7.4 |
| 40 | Isosorbide monooleate | 92.6 | 50:50 | 7.4 |

The upper and/or lower limits of the numerical ranges described in the present description may be arbitrarily combined to define preferred ranges. For example, an upper limit and a lower limit of numerical ranges may be arbitrarily combined to define a preferred range, upper limits of numerical ranges may be arbitrarily combined to define a preferred range, or lower limits of numerical ranges may be arbitrarily combined to define a preferred range.

The present embodiments have been described in detail, but the specific configuration is not limited to these embodiments. Any design changes within the gist of the disclosure are included in the present disclosure.

All publications, patents, and patent applications cited in the description are incorporated herein by reference in their entirety.

## Claims

**1.** A non-lamellar liquid crystal-forming composition comprising:

a fatty acid ester; and
a phospholipid, wherein
the fatty acid ester has a hydrophilic group having one hydroxyl group or a salt thereof, and
the fatty acid ester is a fatty acid ester having none of structures represented by General Formulas (A) to (D):

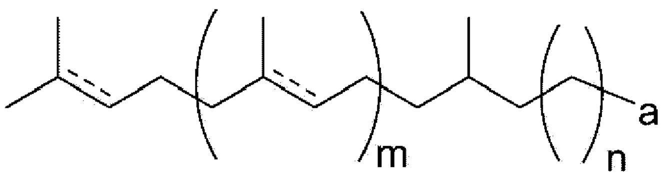

· · · (A)

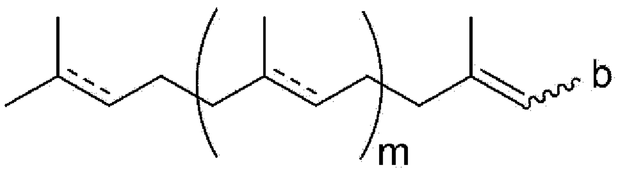

· · · (B)

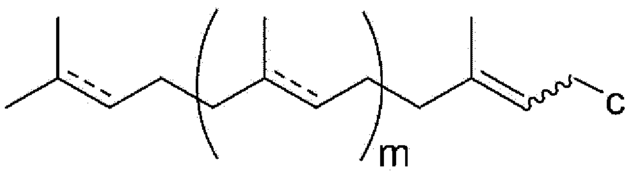

· · · (C)

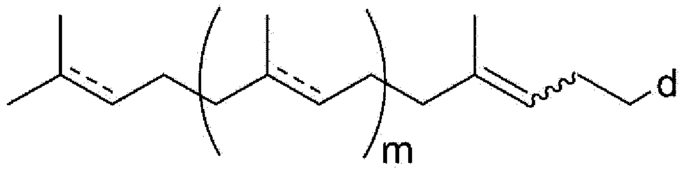

· · · (D)

in General Formulas (A) to (D), m is independently 1 or 2; a, b, c, and d are each independently a binding site to a hydrophilic group;
- - - independently means a single bond or a double bond; in General Formula (A), n is 0, 1, or 2; and in General Formulas (B), (C), and (D), a wavy line independently means an E-isomer or a Z-isomer.

**2.** The composition according to claim 1, wherein the fatty acid ester has a fatty acid having 6 to 24 carbon atoms.

**3.** The composition according to claim 1 or 2, wherein a weight ratio between the fatty acid ester and the phospholipid is 90:10 to 20:80.

**4.** The composition according to any one of claims 1 to 3, wherein the fatty acid ester is a fatty acid ester of at least one selected from a saturated or unsaturated linear fatty acid and a derivative thereof and at least one selected from a glycol and a derivative thereof.

**5.** The composition according to claim 4, wherein the linear fatty acid is a linear fatty acid having an unsaturation degree of 0 to 6.

**6.** The composition according to claim 4 or 5, wherein the linear fatty acid is a linear fatty acid represented by General Formula (I):

$$H_3C\text{-}R\text{-}COOH \quad ... \qquad (I)$$

in General Formula (I), R is a linear hydrocarbon group represented by $-C_pH_q-$; p is an integer of 4 to 22; and q is an integer of 2p - 4 to 2p when p is 5 or less, is an integer of 2p - 6 to 2p when p is 6 or 7, is an integer of 2p - 8 to 2p when p is 8 or 9, is an integer of 2p - 10 to 2p when p is 10 or 11, or is an integer of 2p - 12 to 2p when p is not less than 12.

**7.** The composition according to any one of claims 4 to 6, wherein the linear fatty acid is at least one fatty acid selected from caprylic acid, lauric acid, myristic acid, palmitic acid, oleic acid, linoleic acid, and behenic acid.

**8.** The composition according to any one of claims 4 to 7, wherein the glycol is at least one glycol selected from propylene glycol, ethylene glycol, butylene glycol, 3-methyl-1,3-butanediol, diethylene glycol, and isosorbide.

**9.** The composition according to any one of claims 1 to 8, wherein the phospholipid is at least one phospholipid selected from phosphatidylcholine, phosphatidylethanolamine, and a salt thereof.

**10.** The composition according to any one of claims 1 to 9, wherein the phospholipid is at least one phospholipid selected from soybean phosphatidylcholine, egg yolk phosphatidylcholine, dimyristoyl phosphatidylcholine, dioleyl phosphatidylcholine, dioleyl phosphatidylethanolamine, and a salt thereof.

**11.** The composition according to any one of claims 1 to 10, further comprising at least one of an oil and an organic solvent.

**12.** The composition according to any one of claims 1 to 11, wherein the composition further comprises an aqueous medium and is a non-lamellar liquid crystal composition.

**13.** The composition according to claim 12, wherein the composition further comprises a surfactant and is a non-lamellar liquid crystal emulsion composition.

**14.** The composition according to any one of claims 1 to 11, wherein the composition is a liquid crystal precursor composition capable of forming a non-lamellar liquid crystal in the presence of an aqueous medium.

**15.** A pharmaceutical formulation comprising the composition according to any one of claims 1 to 14.

**16.** The pharmaceutical formulation according to claim 15, for adhesion prevention of living tissue.

**17.** The pharmaceutical formulation according to claim 15, wherein the composition further comprises a drug and is a sustained release formulation.

**18.** The pharmaceutical formulation according to claim 17, wherein the drug is a gonadotropin-releasing hormone (GnRH) agonist.

**19.** The pharmaceutical formulation according to claim 18, wherein the GnRH agonist is leuprolide or a salt thereof.

**20.** The pharmaceutical formulation according to any one of claims 15 to 19, wherein the pharmaceutical formulation is a spray formulation, an aerosol formulation, an injection, or a depot formulation.

**21.** A compound represented by Formula (X') or a salt thereof:

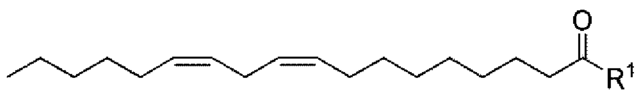 · · · (X' )

in General Formula (X'), $R^1$ is a structure derived from propylene glycol, butylene glycol, isoprene glycol, diethylene glycol, or isosorbide.

**22.** A compound represented by Formula (X), (Y), or (Z):

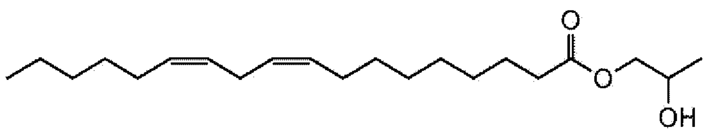 · · · (X)

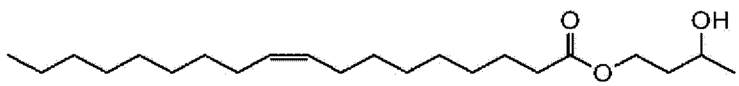 · · · (Y)

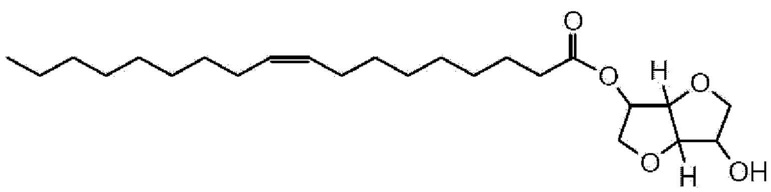 · · · (Z)

or a salt thereof.

Fig. 1

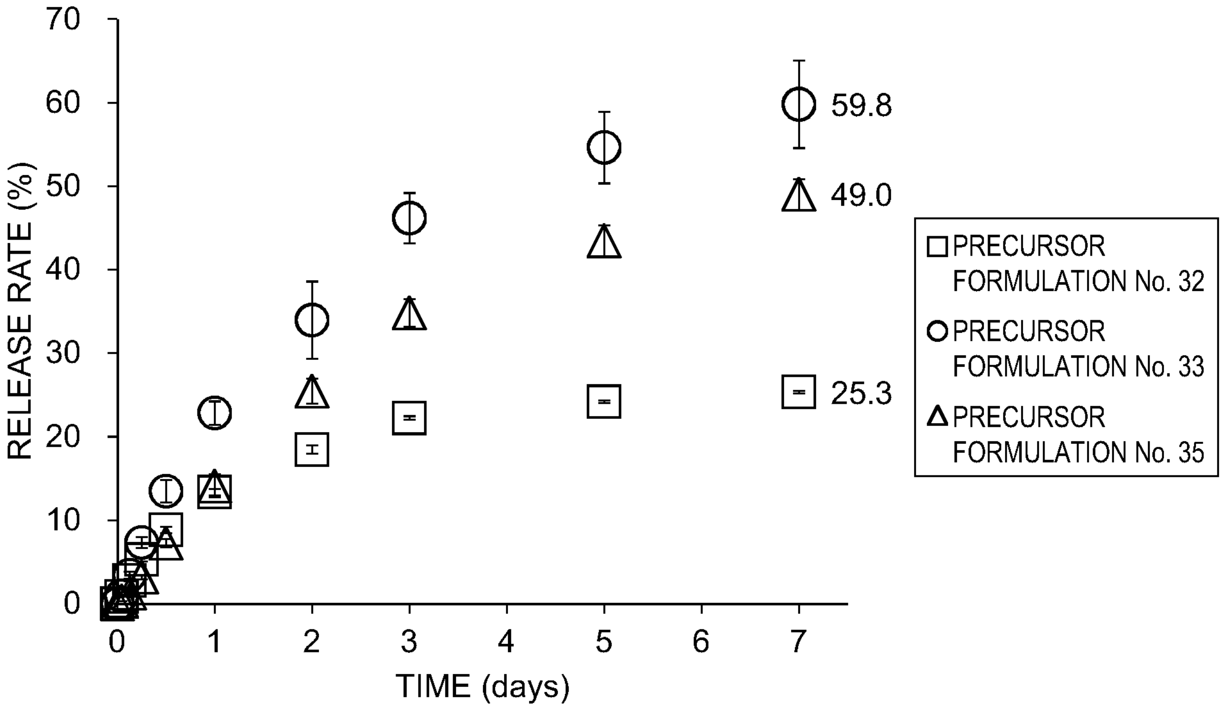
RELEASE RATE (%)
70
60
50
40
30
20
10
0
0
1
2
3
4
5
6
7
TIME (days)
59.8
49.0
25.3
PRECURSOR FORMULATION No. 32
PRECURSOR FORMULATION No. 33
PRECURSOR FORMULATION No. 35

Fig. 2

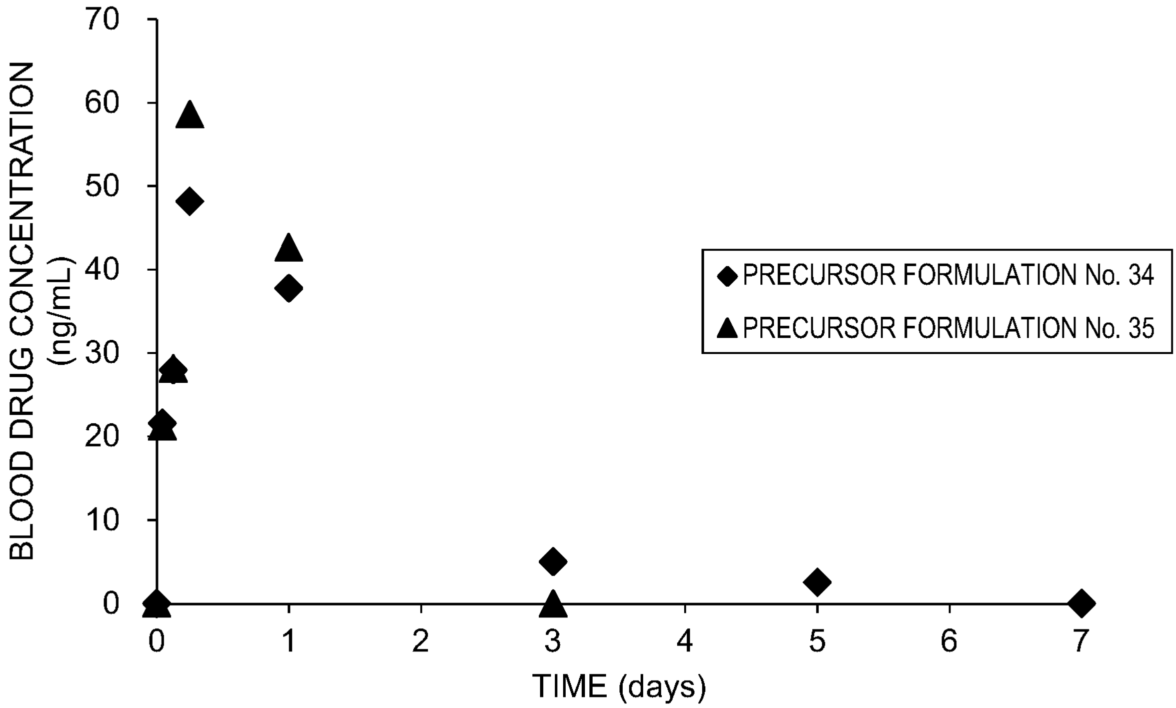
BLOOD DRUG CONCENTRATION (ng/mL)
70
60
50
40
30
20
10
0
0
1
2
3
4
5
6
7
TIME (days)
PRECURSOR FORMULATION No. 34
PRECURSOR FORMULATION No. 35

Fig. 3

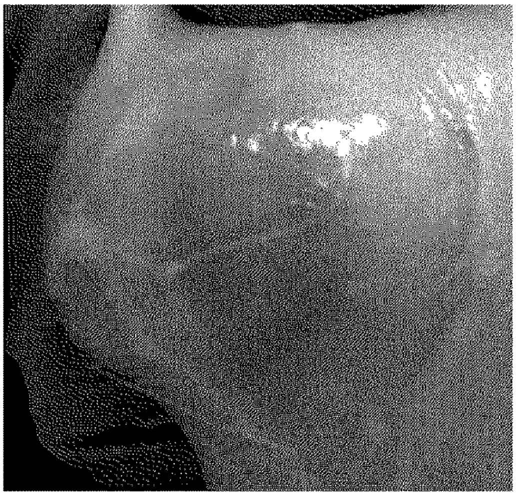

**INTERNATIONAL SEARCH REPORT**

International application No.
**PCT/JP2022/029014**

**A. CLASSIFICATION OF SUBJECT MATTER**

***A61K 9/107***(2006.01)i; ***A61K 9/12***(2006.01)i; ***A61K 38/09***(2006.01)i; ***A61K 45/00***(2006.01)i; ***A61K 47/14***(2006.01)i; ***A61K 47/24***(2006.01)i; ***A61P 13/08***(2006.01)i; ***A61P 35/00***(2006.01)i
FI: A61K9/107; A61K47/14; A61K47/24; A61K9/12; A61K45/00; A61K38/09; A61P35/00; A61P13/08

According to International Patent Classification (IPC) or to both national classification and IPC

**B. FIELDS SEARCHED**

Minimum documentation searched (classification system followed by classification symbols)

A61K9/00-33/44, A61K47/00-47/69

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2015-505833 A (CAMURUS AB) 26 February 2015 (2015-02-26) claims, paragraphs [0001], [0057], [0058], [0094] | 1-3, 9-12, 14-15, 17-20 |
| Y | | 13, 16 |
| A | | 4-8 |
| X | JP 10-504532 A (GS DEVELOPMENT AB) 06 May 1998 (1998-05-06) claims, example 4 | 1-3, 9-12, 14-15, 17, 20 |
| Y | | 13, 16 |
| Y | WO 2014/178256 A1 (FARNEX INCORPORATED) 06 November 2014 (2014-11-06) claims 1, 9, paragraphs [0046], [0090] | 13, 16 |
| X | CN 105613493 A (SHENZHEN BIOGLOBAL AGRICULTURAL SCIENCE CO LTD) 01 June 2016 (2016-06-01) example 8 | 21, 22 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 August 2022** | **06 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/029014**

**C. DOCUMENTS CONSIDERED TO BE RELEVANT**

| Category* | Citation of document, with indication, where appropriate, of the relevant passages | Relevant to claim No. |
|---|---|---|
| X | WO 2020/070846 A1 (TAKEMOTO YUSHI KABUSHIKI KAISHA) 09 April 2020 (2020-04-09) paragraph [0057] | 22 |
| X | US 2018/0155357 A1 (BASF SE) 07 June 2018 (2018-06-07) paragraphs [0010], [0014], [0024], [0025] | 22 |

Form PCT/ISA/210 (second sheet) (January 2015)

**INTERNATIONAL SEARCH REPORT**

International application No.
**PCT/JP2022/029014**

**Box No. III Observations where unity of invention is lacking (Continuation of item 3 of first sheet)**

This International Searching Authority found multiple inventions in this international application, as follows:

The inventions in claims 1, 21, and 22 share the common technical feature of a "fatty acid ester that does not have any structures represented by general formulae (A)-(D)."

However, said technical feature does not make a contribution over the prior art in light of the disclosure of documents (JP 2015-505833 A (CAMURUS AB) 26 February 2015 (2015-02-26), claims, paragraphs [0057], [0058], JP 10-504532 A (GS DEVELOPMENT AB) 06 May 1998 (1998-05-06), claims, CN 105613493 A (SHENZHEN BIOGLOBAL AGRICULTURAL SCIENCE CO LTD) 01 June 2016 (2016-06-01), example 8, WO 2020/070846 A1 (TAKEMOTO YUSHI KABUSHIKI KAISHA) 09 April 2020 (2020-04-09), paragraph [0057], US 2018/0155357 A1 (BASF SE) 07 June 2018 (2018-06-07), paragraphs [0010], [0014], [0024], [0025]), and thus cannot be considered a special technical feature. Furthermore, there are no other same or corresponding special technical features between these inventions.

Also, the claims include the following three inventions (groups).

(Invention 1) Invention in claims 1-20

A "non-lamellar liquid crystal-forming composition comprising: a fatty acid ester and a phospholipid, wherein the fatty acid ester has a hydrophilic group having one hydroxyl group or a salt thereof, and the fatty acid ester does not have any structures represented by general formulae (A)-(D)."

(Invention 2) Invention in claim 21

A "compound represented by formula (X') or a salt thereof."

(Invention 3) Invention in claim 22

A "compound represented by formula (X), (Y), or (Z) or a salt thereof."

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/JP2022/029014**

| Patent document cited in search report | Publication date (day/month/year) | Patent family member(s) | Publication date (day/month/year) |
|---|---|---|---|
| JP 2015-505833 A | 26 February 2015 | US 2014/0348903 A1<br>claims, paragraphs [0001], [0091], [0092], [0132]<br>WO 2013/083460 A1<br>CN 104105479 A<br>KR 10-2014-0111661 A | |
| JP 10-504532 A | 06 May 1998 | US 5807573 A<br>claims, example 4<br>WO 1995/034287 A1 | |
| WO 2014/178256 A1 | 06 November 2014 | US 2016/0067208 A1<br>claims 1, 9, paragraphs [0160], [0204]<br>EP 2992910 A1<br>CN 105358190 A | |
| CN 105613493 A | 01 June 2016 | (Family: none) | |
| WO 2020/070846 A1 | 09 April 2020 | US 2021/0355291 A1<br>paragraph [0084]<br>EP 3862392 A1<br>CN 112789318 A | |
| US 2018/0155357 A1 | 07 June 2018 | WO 2016/169833 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014527545 A
- JP 2018502091 A
- JP 2021122489 A